# EUROPEAN PATENT SPECIFICATION

(11) **EP 1 439 776 B1**
(45) Date of publication and mention of the grant of the patent: **16.06.2010**
(21) Application number: 02773035.7
(22) Date of filing: 31.10.2002
(51) Int. Cl.: A61B 1/267, A61M 16/04

(54) **INSTRUMENT FOR CREATING SPACE IN A HUMAN PHARYNX**
INSTRUMENT ZUR VERSCHAFFUNG VON RAUM IN EINEM MENSCHLICHEN MUNDKEHLLOCH
INSTRUMENT DE CREATION D'UN ESPACE DANS LE PHARYNX HUMAIN

(30) Priority: 31.10.2001 NL 1019263; 12.12.2001 NL 1019547; 12.12.2001 NL 1019550
(43) Date of publication of application: 28.07.2004
(73) Proprietor: Larynguide B.V., 3011 AA Rotterdam (NL)
(72) Inventor: Tjong Joe Wai, Peter, 1017 VX Amsterdam (NL)
(74) Representative: Swinkels, Bart Willem
(86) International application number: PCT/NL2002/000692
(87) International publication number: WO 2003/051186

(56) References cited:
- DE-A- 3 007 994
- FR-A- 2 272 632
- GB-A- 2 258 398

## Description

The present invention relates in general terms to an instrument for creating space in a human pharynx, which leads to the space between the rear side of the pharynx and the tongue of a person being widened, so that the airway is widened.

An instrument of this type is, for example, a Guedel airway kit or a Berman airway kit which are known from practical use and are intended to widen the airway.

An instrument of this type is formed as a hollow tube which is bent into a hook shape and functions as an insertion part with an insertion end. At a free end of the known airway kit there is a closure plate which is substantially at right angles thereto. During use, the insertion part of the known airway kit is guided along the tongue of the person and into the pharynx. While the airway kit is being used, the closure plate remains outside the mouth of a person and prevents the airway kit from disappearing into the pharynx. In the introduced state, the hollow tube is located between the tongue and the rear side of the pharynx and creates a passage allowing air to reach the lungs. Furthermore, to assist breathing in general, an artificial respiration mask is placed over the nose and mouth of a person and the artificial respiration air flows through the airway kit or hollow tube to the lungs.

The known instrument is used if a person can no longer breathe independently or needs help with breathing, for example during an operation or after an accident in which the person has lost consciousness. At such a time, it is necessary for breathing to be restored as quickly as possible and in any event to be assisted. As soon as someone has been placed under anaesthetic, for example during an operation, or has become comatose, it is a matter of life and death that a clear airway be ensured. The unconscious person is no longer able to breathe of his own accord and the muscles of the pharynx become weak. As a result, the tongue sinks backwards into the pharynx and blocks the airway. In such a situation, the known instrument clears the airway to some extent.

However, one drawback of the known instrument is that in practice it does not offer sufficient reliability with regard to keeping clear access to the lungs and therefore cannot ensure a clear airway in all situations.

Therefore, it is an object of the present invention to provide an instrument which at least partially eliminates the abovementioned drawbacks or at least to provide an alternative to the known instrument.

In particular, it is an object of the present invention to provide an instrument for creating space in the pharynx, so that the airway is widened.

This object is achieved by an instrument as claimed in claim 1.

This combination of measures provides an instrument which can easily be introduced into the pharynx and can follow the anatomy of the pharynx and the contour of the tongue, and with which, on account of the fact that the basic element and the spacer can be displaced with respect to one another, space can be created in the pharynx. As a result, the distance between the tongue and the rear side of the pharynx is increased and the airway will be cleared with a high degree of reliability. As a result it is, for example, easy to supply artificial respiration air to the pharynx or to position an object, such as an artificial respiration tube, in the pharynx. It is particularly advantageous that space is created in the pharynx without forces having to be exerted from outside, and in fact forces are only active between the rear side of the pharynx and the tongue.

Moreover, when the instrument is being introduced, it will be suitably guided over the tongue and the instrument can reliably be correctly positioned in the pharynx. Moreover, in the introduction and holding-open position, the follower part closely follows the contour of the tongue, so that the tongue can as far as possible be kept away from the rear side of the pharynx.

It is noted that FR 2272632 discloses a laryngoscope, which is provided with a mirror for viewing into a bronchus of a person.

It is also noted that DE 3007994 describes a guiding device having an outer guiding tube and an inner guiding tube. The guiding tubes are bent at their distal ends and are slidably connected to each other. After the guiding device has been introduced into the bronchus of a person, a catheter can be guided through the inner tube. Then, the curvature of the catheter is adjusted by sliding the guiding tubes with respect to each other.

It is furthermore noted that GB 2258398 discloses a laryngoscope comprising a beak for insertion into a patient's throat and a moveably mounted plate carried on the beak. The plate can be closed onto the free end of the beak during insertion thereof into the throat but is movable away from the free end so as to displace any soft tissue in the patient's throat and improve visibility at the distal end of the beak. The plate is provided with a mirror on the side facing the beak.

A further drawback of the known instrument is that the position in the pharynx where the airflow from the artificial respiration apparatus arrives is located close to the entry to the trachea and is impeded by the epiglottis.

In a preferred embodiment, the follower part extends beyond the insertion end of the insertion part.

This leads to the introduction of the instrument ending as a result of the end of the follower part being the first piece to reach the angle which is included by the epiglottis and the base of the tongue, before the basic element can touch the epiglottis. When the instrument is opened up, the follower part will, as it were, latch securely into this angle so that the instrument can no longer easily be forced out of the pharynx. However, what is more important is that the epiglottis, which closes the trachea when a person swallows something, is suitably folded away as a result of the basic element and the spacer being displaced away from one another, as a result of the displacement of the tongue, without there being any possibility of the insertion end of the insertion part, which is at a higher level, causing an obstacle. The space in the pharynx and in particular the access to the trachea is completely or at least partially cleared.

Moreover, the person who is handling the instrument outside the pharynx, just above the larynx, can feel that the instrument has been placed in the correct position, since the end of the follower part extends beyond the insertion end of the insertion part. In this way, it is easy to check whether the instrument has been introduced correctly.

In a further preferred embodiment, the follower part and/or the insertion part extends substantially through an angle of 90°, so that when the basic element and the spacer are displaced with respect to one another from the introduction position into the holding-open position, the instrument can remain hooked even more securely behind the tongue and cannot easily be removed from the pharynx.

In a further advantageous variant, the insertion part is made at least in part from a deformable material, in particular an elastically deformable material. The fact that at least the curved insertion part of the spacer is deformable, and in particular elastically deformable, opens up the possibility of altering the curvature or radius of the insertion part. This results in an instrument which makes it possible to adjust the direction in which the basic element is oriented with respect to the pharynx and allows the space which is present therein to be utilized optimally. If the instrument is used to supply artificial respiration air to a person, in this way the flow of artificial respiration air can moreover be directed as successfully as possible toward the entry to the trachea.

In a preferred embodiment, the instrument is provided with locking means for locking the basic element and the spacer at least in the holding-open position. This prevents the opened instrument from being pushed back into a closed position by the forces exerted on it by the rear wall of the pharynx and the soft parts in the pharynx. Moreover, the person who is handling the instrument does not have to continue to exert a force on the instrument in order to hold it in the holding-open position, so that he has his hands free to carry out other actions.

In a further preferred embodiment, the spacer and the basic element comprise cooperating guide means, and in particular the spacer and the basic element are provided with guide means which engage with one another in complementary fashion. This ensures simple and reliable guidance of the basic element in and/or along the spacer.

In a further preferred embodiment, the basic element and the spacer, in the introduction position, substantially adjoin one another and, in the introduction position, have a combined thickness which is substantially equal to the maximum thickness of the basic element and/or the spacer. This creates an instrument which can be introduced into the pharynx of a person, even if this person can scarcely open his mouth, for example in the event of trauma.

In a preferred embodiment, the spacer comprises at least two limbs which are positioned at a distance from one another, and in particular the limbs form an open structure for receiving the basic element in the spacer in order to allow displacement of the basic element through the spacer. As a result, the thickness of the instrument is reduced still further, and the extent to which the person has to open his mouth can be reduced still further for successful introduction of the instrument.

In a variant of the instrument, the basic element comprises guide channels for receiving the limbs in such a manner that they are guided therein, so that further improved guidance of the basic element through the spacer is achieved.

In a preferred embodiment, the basic element delimits a through-flow passage for guiding artificial respiration air, so that the artificial respiration air can be supplied to the pharynx in very targeted fashion and can be supplied to the trachea in very targeted fashion. In this case, the fact that the basic element and the spacer can be displaced with respect to one another ensures that in the holding-open position the basic element opens out in the vicinity of the rear side of the pharynx and, from there, supplies the artificial respiration air to the pharynx, resulting in improved supply to the trachea.

In a further variant of the instrument, the spacer is provided, at a free end, with a first delimiting body for limiting the insertion depth of the spacer. This makes it possible to prevent the spacer, during introduction of the instrument, from being pushed too far into the mouth of a person and being completely inside the mouth and/or pharynx.

In a further variant of the instrument, the basic element is provided, at a free end, with a second delimiting body for limiting the insertion depth of the basic element. During movement of the basic element along the spacer, the second delimiting body will come to a standstill against the first delimiting body, and in this way it is possible to prevent the basic element from being introduced too far into the pharynx.

In yet another variant of the instrument, the first delimiting body comprises at least one through-opening for supporting at least a section of the basic element in such a manner that it is guided therein, resulting in simple guidance and coupling between the basic element and the spacer.

As is known, breathing and swallowing take place along different passages which each start at the back of the pharynx. One of these passages, the trachea, extends from the pharynx into the lungs, while the other passage, the oesophagus, extends into the stomach.

If, as described above, a person is no longer able to breathe independently and it is necessary for breathing to be restored as quickly as possible and in any event to be supported, another procedure, which is likewise in very widespread use, is that of introducing an artificial respiration tube into the trachea of the person and supplying air to the person in question via this artificial respiration tube: a procedure known as intubation.

To allow reliable positioning of an artificial respiration tube in the trachea, it is desirable for the person carrying out the intubation to be able to see the opening of the trachea. However, there is a considerable angle between the respective axes of the mouth and the larynx, and moreover the tongue and the epiglottis are also situated between the mouth and the opening of the trachea, and during swallowing the epiglottis closes the larynx, so that it is impossible for any food to enter the trachea. These circumstances make it impossible to see the larynx via the mouth. Partly on account of the fact that the openings of the trachea and the oesophagus are very close together, it is virtually impossible to place an artificial respiration tube in the trachea without any risk of the artificial respiration tube which is to be positioned in the trachea unintentionally "disappearing" in the oesophagus.

An intubation method which is in widespread use in practice uses a laryngoscope as an auxiliary tool. The known laryngoscope comprises a handle, at one end of which a curved part or blade is fitted at right angles and is intended to be introduced into the pharynx of the person. The person carrying out the intubation stands at the head end of the person who is to be intubated and introduces the blade into the mouth with an end of the curved part directed towards the trachea. In the process, the curved part or blade slides along the tongue and, as a result of a rotary movement, the blade of the laryngoscope is moved further into the pharynx until its end rests in an angle which is included between the epiglottis and the tongue. Then, the laryngoscope is pulled upwards by the handle, so that the tongue is pressed upwards out of the field of view of the person carrying out the intubation. During this tilting and pushing movement, the head of the person who is undergoing the intubation is tilted backwards and even lifted, and the person carrying out the intubation can see the opening of the trachea via the open mouth and can then introduce the artificial respiration tube into it. Therefore, with this method it is possible to make the opening of the trachea visible, so that the person who is to carry out an intubation can see where he is working. However, this method has a large number of drawbacks.

In the abovementioned procedure, considerable forces have to be exerted on the head of the person who is to be intubated in order to sufficiently tilt the head to provide a good view of the pharynx. This entails the risk of damage to, for example, vertebrae of the neck of the person. In addition, there is a risk of broken teeth and damaged teeth roots, since the rigid blade of the laryngoscope is pressed onto the teeth while the head is being lifted and the opening of the trachea is being exposed. There is also likely to be damage to the soft parts located in the pharynx.

In addition, some people are unable to open their mouth sufficiently far to allow successful introduction of the blade of the laryngoscope into the mouth. In this case, additional force has to be exerted in order to open the mouth sufficiently, which in turn entails a risk of broken teeth or other forms of trauma. Moreover, with the method described above, it is necessary for the person carrying out the intubation to be very well trained and have considerable experience if the intubation is to be carried out successfully. In addition, another person's assistance is generally required, for example to hold the head of the person who is to be intubated or carry out other duties in order to allow intubation to be carried out successfully.

A final drawback of the known instrument is that it gives poor results for people with an unusual anatomy of the pharynx or jaw. In such a situation, it is very difficult to carry out successful intubation using a standard laryngoscope.

A preferred embodiment of the instrument according to the present invention is **characterized in that** the basic element and the insertion part are designed to guide a tubular object over at least a section of an inner surface of the basic element and the insertion part, and at least partially eliminate these drawbacks. With this embodiment, it is not necessary for the person handling the instrument to have a clear view of the pharynx to allow successful introduction of the object. The fact that the basic element and the spacer can be displaced with respect to one another, in particular in the pharynx, ensures that a play of forces inside the pharynx creates space, instead of the standard lifting and pushing movements from outside, as required with the abovementioned laryngoscope. Moreover, the play of forces occurring within the pharynx has the advantage that no forces, or at least greatly reduced forces, are then exerted on the teeth and on the neck.

In a further preferred embodiment, there are means for moving the instrument into the introduction position, in which the inner surface of the insertion part rests on the follower part and in which the follower part and the insertion part have substantially the same radius. Furthermore, there are means for moving the instrument into the holding-open position, in which the insertion part is at a distance from the follower part and in which the insertion part has a smaller radius than the follower part.

Making at least the curved insertion part of the spacer deformable, and in particular elastically deformable, offers the option of altering the curvature or radius of the curved insertion part. The result of this is an instrument with which it is possible to introduce a substantially tubular object into the trachea of a person with a very high level of reliability.

In the introduced state, the outwardly directed surface of the insertion part can rest against the rear side of the pharynx. As a result of the insertion part being moved onwards into the pharynx from this position, the curvature of the insertion part will change on account of the counterpressure exerted on the insertion part by the rear wall of the pharynx. It is therefore possible to adjust the angle at which the object to be introduced, for example and artificial respiration tube, is introduced, so that the entry of the oesophagus cannot be reached by the artificial respiration tube. By increasing the curvature, i.e. reducing the radius, the object will, as it were, arrive more from the rear side of the head of the person. If the curvature is not increased, the object will, as it were, arrive more from the top of the head of the person. It is therefore possible to approach the trachea as ideally as possible, and it is also possible to adjust the direction with which the abovementioned object approaches the larynx to the prevailing conditions. More particularly, increasing the curvature of the insertion part means that an object which is to be introduced will move past the entry to the oesophagus so that it can be guided into the trachea.

In an advantageous variant of the instrument, the insertion part, in the closed position, rests with prestress on the follower part. In the open position, the insertion part is substantially stress-free. This creates an instrument in which the insertion part has a "precurvature", i.e. the insertion part has a starting position with a curvature which is greater than the curvature of the follower part. Moving the insertion part and the follower part apart results in the insertion part adopting its "natural" starting position, so that it will automatically adopt a more curved position than the follower part.

In a preferred embodiment, there are coupling means for the limited and displaceable coupling of the basic element and the spacer, the coupling means being arranged in the vicinity of a free end of the follower part and in the vicinity of a free end of the insertion part. In this way, the displaceability of the basic element with respect to the spacer is limited and has its effect in the vicinity of the free ends of the follower part and the basic element. As a result, when a substantially longitudinally oriented force is exerted on the basic element, from the moment at which the displacement thereof is limited, the insertion part will be deformed, since its free end cannot move any further.

In an advantageous variant, the coupling means are at least in part made from a flexible material, so that the coupling means will not impede displacement of the insertion part with respect to the follower part.

In a further variant, the spacer comprises a support member which acts as a tilting point for the basic element, with in particular an outwardly directed surface of the basic element bearing freely against the said support member. As a result, the basic element can execute a tilting or pivoting movement with respect to the spacer. The result of this is in particular that the free end of the insertion part can execute a movement with respect to the free end of the follower part, specifically in a direction which is substantially at right angles to the longitudinal direction. This offers a further possibility for adjusting the direction in which an object approaches the larynx to the prevailing conditions.

In an advantageous variant of the instrument according to the invention, the spacer comprises two limbs which are positioned at a distance from one another, for at least partially receiving the basic element between them in the spacer, and there is a spreading mechanism for increasing the distance between the limbs. This makes it possible for the basic element to be taken away separately from the spacer, between the limbs, so that the basic element and the spacer can be removed from the pharynx separately from one another.

Characteristic features and advantages of the present invention will be explained in more detail with reference to the following description of preferred embodiments of an instrument and a method according to the invention with reference to the drawing, in which identical reference numerals denote identical or similar components, and in which:
Figure 1 shows a side view of an instrument according to the invention;
Figure 2 shows a plan view of the instrument shown in Figure 1;
Figure 3 shows a side view of the instrument shown in Figure 1 in an introduced position thereof;
Figure 4 shows a side view of the instrument shown in Figure 1 in an introduced and opened position thereof;
Figure 5 shows an alternative embodiment of the instrument according to the invention;
Figure 6 shows a diagrammatic, perspective view of a free end of the instrument shown in Figure 5;
Figure 7 shows a cross section on line II-II in Figure 5 through the instrument shown in Figure 5;
Figure 8 shows a diagrammatic, perspective view of locking means for use with the instrument shown in Figure 5;
Figures 9A-9C show an alternative embodiment of the instrument shown in Figure 1;
Figures 10A-10C show an alternative embodiment of the instrument shown in Figures 9A-9C;
Figure 11 shows a perspective view of a variant of an instrument according to the invention;
Figure 12 shows a side view of the instrument shown in Figure 11 just before it is introduced;
Figure 12A shows a cross section on line IIa-IIa in Figure 12 through the instrument shown in Figure 12;
Figure 13 shows a side view of the instrument shown in Figure 11 in an introduced position thereof;
Figure 14 diagrammatically depicts a front view of an end of a follower part of the instrument shown in Figure 11;
Figure 15 shows a side view of the instrument shown in Figure 11 in an introduced and opened position thereof;
Figure 16 diagrammatically depicts a side view of the instrument shown in Figure 11 in a tilted position;
Figure 17 diagrammatically depicts a cross section through a variant of the instrument shown in Figure 16;
Figure 18 diagrammatically depicts a longitudinal section through a free end of the basic element;
Figure 19 shows a side view of a variant of the instrument according to the present invention;
Figure 20 shows a diagrammatic and perspective illustration of part of the instrument shown in Figure 19;
Figure 21 shows a diagrammatic cross-sectional view through the instrument shown in Figure 19;
Figure 22 shows a diagrammatic side view of a variant of the instrument according to the invention;
Figure 22A shows a perspective view of the instrument shown in Figure 22;
Figure 23 shows a cross section on line III-III in Figure 22 through the instrument shown in Figure 22;
Figures 24A-24E show the introduction of an artificial respiration tube using the instrument shown in Figure 22;
Figure 25 shows a cross section on line IV-IV in Figure 22 through the instrument 200;
Figure 26 shows a plan view, with the housing removed, of part of the instrument shown in Figure 22, and
Figure 27 shows a variant of the instrument according to the present invention with a spacer which can be split.

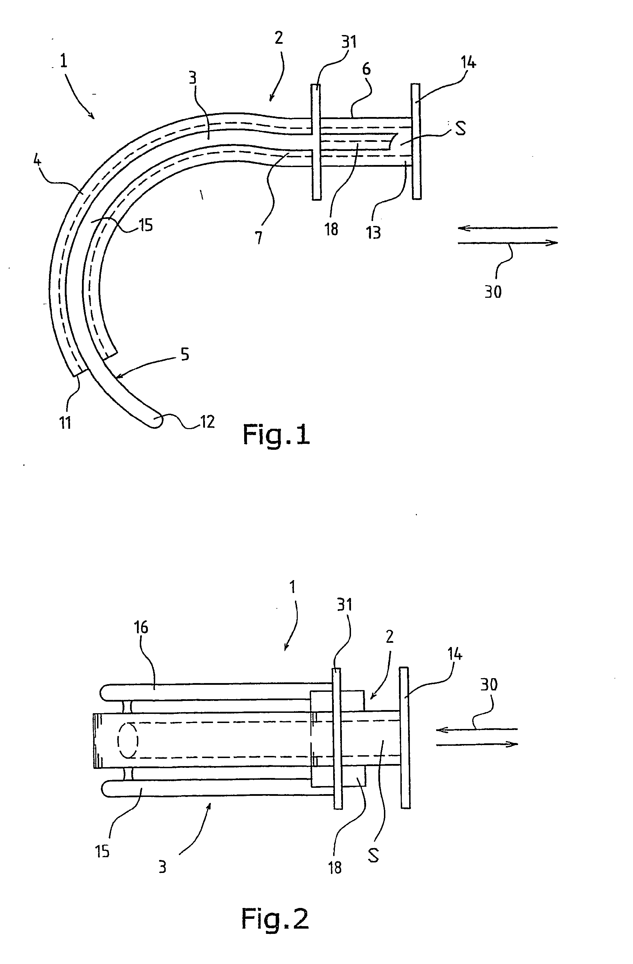

Figure 1 shows an instrument according to the invention, which is denoted overall by reference numeral 1. The instrument 1 comprises a basic element 2 and a spacer 3. The basic element 2 and the spacer 3 are both substantially J-shaped. The basic element 2 comprises a curved insertion part 4 with a free insertion end 11. The spacer 3 comprises a curved follower part 5 with a free end 12. The follower part 5 is designed to follow the contour of a tongue, and the curved follower part 5 preferably extends through an angle of approximately 90°, preferably through an angle of more than 90°. However, this angle is not highly critical. It is important that the angle is such that, as will be explained more clearly below, the follower part 5 will remain securely in the pharynx of a person during use of the instrument. Furthermore, the basic element 2 and the spacer 3 each comprise a substantially straight section 6 and 7, respectively, adjoining the insertion part 4 and the follower part 5, respectively. The straight section 7 of the spacer 3 is in this case very short, but may also be longer.

In the embodiment shown, the basic element 2 delimits a through-flow passage S, as diagrammatically indicated by dashed lines in the figure, in order to guide artificial respiration air into a pharynx of a person. The straight section 6 of the basic element 2 comprises an end 13 at which a first delimiting body 14 is arranged substantially at right angles to the straight section 6. The first delimiting body 14 may, for example, comprise a closure plate or another suitable body which closes off the mouth of a person when the instrument 1 is being used. In the first delimiting body 14 there is a through-opening (not shown in the figure) which is connected to the interior of the through-flow passage S.

The spacer 3 has an open structure and is preferably formed as a substantially J-shaped framework, for example made from a tubular material. The open structure of the spacer 3 is delimited by a distance between two substantially parallel limbs 15 and 16 of the framework. Only one of the limbs 15 can be seen in the figure.

The dimensions in the transverse direction of the basic element 2 are smaller than the distance between the limbs 15, 16 of the spacer 3, and therefore the basic element 2 fits into the open structure of the spacer 3, and it is possible for the basic element 2 to move through spacer 3. For this purpose, the spacer 3 is likewise provided, at a free end, with a second delimiting body 31. In the second delimiting body 31, there is a through-opening for allowing movement of the basic element 2 and supporting the basic element 2 so that it is guided in the spacer 3. This is diagrammatically indicated by a double arrow 30.

Furthermore, the basic element 2 is provided, on both sides of the straight section 6, with guide channels 18, in which the limbs 15, 16 are at least partially accommodated. This ensures reliable guidance of the basic element 2 in the spacer 3. However, it is also possible not to use any guide channels.

Figure 2 shows a plan view of the instrument 1. As is clearly apparent from the figure, the basic element 2 lies between the limbs 15, 16 of the spacer 3. Dashed lines once again indicate that the basic element 2 delimits a through-flow passage S. In the embodiment shown, the spacer 3 is formed from a curved wire-like or tubular material, such as a thin metal tube or a solid flexible metal wire, which is bent substantially into a J shape. Furthermore, it is clearly apparent that the basic element 2 projects through the delimiting body 31. This makes it possible to minimize the combined thickness of the instrument 1.

The first delimiting body 14 and the second delimiting body 31 preferably have a substantially elliptical contour, but may also be of some other shape, for example may be round or square. The substantially elliptical contour is preferred, however, since this shape means that there are no sharp corners which could damage the mouth of a person. Also, an ellipse shape matches that of the closed mouth of a person.

In the configuration shown in Figures 1 and 2, the basic element 2 is located in the open structure of the spacer 3 and therefore projects through the spacer 3. Since the basic element 2 is located between the limbs 15 and 16 of the spacer 3 and the basic element 2 at least partially projects through the through-opening, lateral displacement of the basic element 2 with respect to the spacer 3 is prevented. However, displacement along the straight section 7 of the spacer 3 is possible. This is indicated by a double arrow 30 in Figures 1 and 2.

The use of the instrument according to the invention will be explained in more detail in the following description.

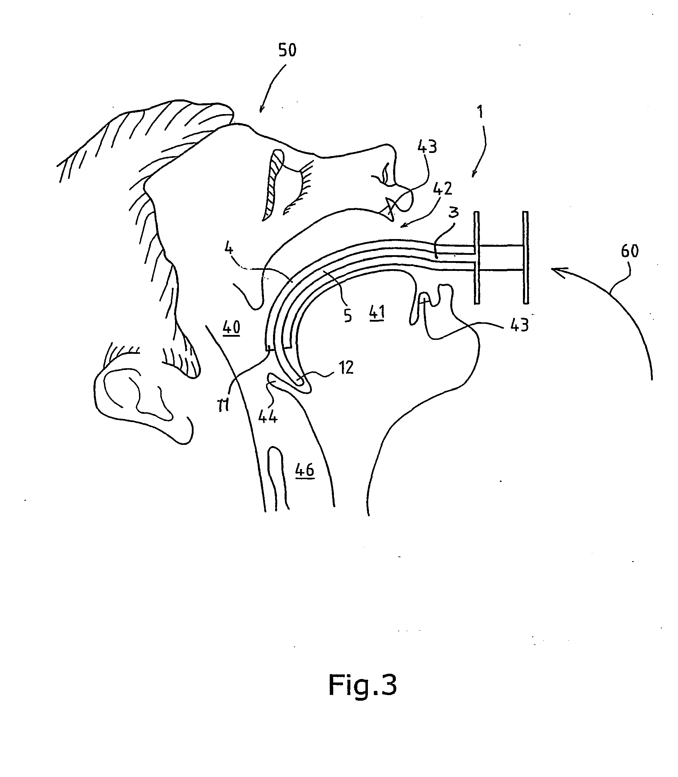

The introduction position or starting position of the instrument 1 before it has been introduced into a pharynx 40 of a person is the position shown in Figure 1. The basic element 2 lies largely between the limbs 15, 16 of the spacer 3. From the introduction position shown in Figure 1, the instrument 1 can be introduced into an open mouth of a person. This is shown in Figure 3.

Figure 3 shows the instrument 1 in the most compact position, after it has been introduced into the pharynx 40 of a head 50 (which is only diagrammatically depicted) of a person. The head 50 includes at least a tongue 41, a mouth opening 42, a row of teeth 43 and a trachea 46.

To introduce the instrument 1 into the pharynx 40, the instrument 1 is guided from a position located outside the mouth opening 42 along the surface of the tongue 41 and into the pharynx 40 by means of a rotary movement by the follower part 5. This is indicated by an arrow 60. The guiding of the instrument 1 inwards stops when the free end 12 of the follower part 5 rests in an angle which is included between the tongue 41 and an epiglottis 44. In this way, the introduction of the instrument 1 reaches a natural end, and it is possible to prevent the instrument 1 from being pushed too far into the pharynx 40.

Correct introduction of the instrument 1 can be checked on the outside of the neck of the person by pulling the instrument 1 slightly from the position shown. As a result, it will be possible to feel the end 12 of the follower part 5 as a small lump just above the Adam's apple (not shown) of the person, specifically just below the tongue bone (not shown). If the lump is felt elsewhere or is not felt at all, the instrument 1 is not correctly positioned in the pharynx 40.

As is clearly apparent from the figure, the follower part 5 hooks into the angle between the epiglottis 44 and the tongue 41 in such a manner that the instrument 1 cannot easily be removed from the pharynx 40. The only way in which the instrument 1 can be removed again from the pharynx 40 is by guiding the follower part 5 back over the surface of the tongue 41 by means of a rotary movement (in a clockwise direction in the figure). In this context, it is advantageous for the follower part 5 to extend through an angle of more than 90°. This ensures reliable positioning of the instrument 1 and means that it will not be easy for the instrument 1 to come out of the pharynx 40 during use.

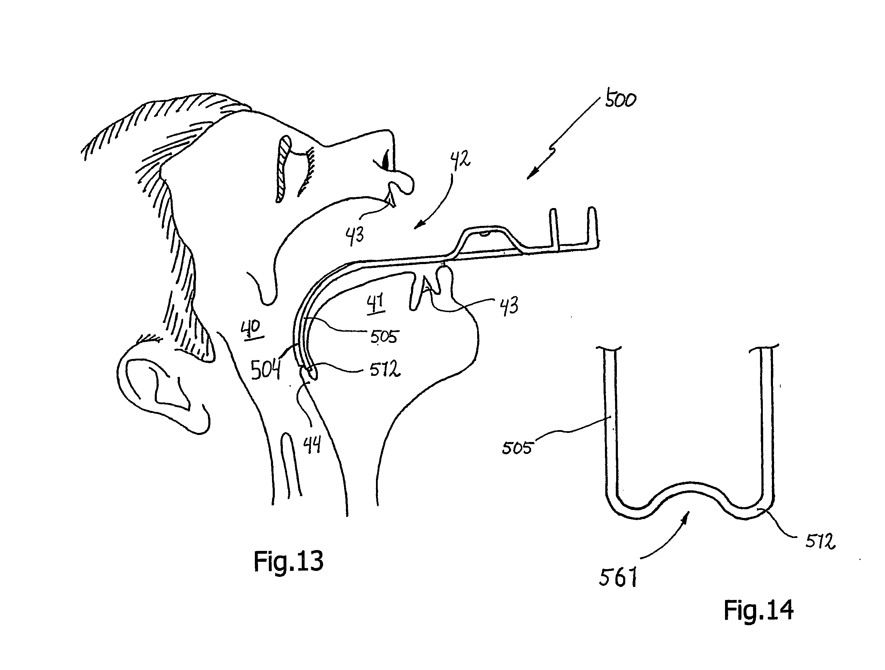

To prevent the free end 12 of the follower part 5 from coming out of the said angle and/or to further simplify positioning of the instrument 1 in the pharynx 40, it is advantageous to provide the free end 12 of the follower part 5 with a recess (cf. for example Figure 13). It is preferable for the recess to be rounded, in order to prevent injury to the tongue 41 and/or the epiglottis 44. However, the recess may also be of any other suitable shape.

Figure 3 shows the instrument 1 in a position in which the instrument is still in the introduction position but in which at least the follower part 5 and the insertion part 4 are located inside the pharynx 40 of the person.

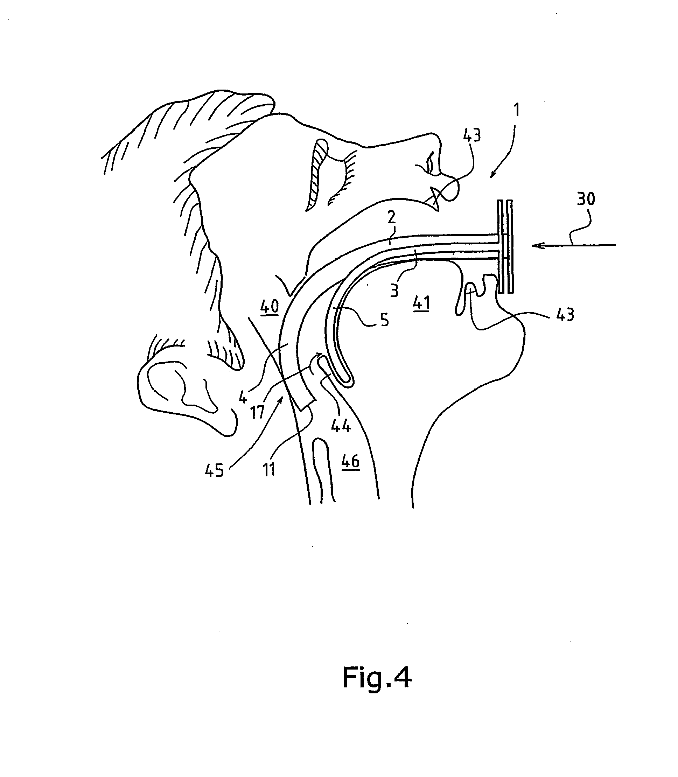

In the position shown in Figure 3, the person has fully opened his mouth 42. However, as can be seen clearly from the figure, there is still a considerable space between the upper teeth and the instrument 1, and it is not necessary for the person to have his mouth 42 fully opened to allow introduction of the instrument 1. The following step of introducing the instrument 1 into the pharynx 40 is explained in more detail with reference to Figure 4.

Figure 4 shows the instrument 1 in an extended or open position. From the introduction position shown in Figure 3, the basic element 2 is displaced with respect to the spacer 3 by the person who is handling the instrument 1, as indicated by means of arrow 30 in the figure.

As a result of the basic element 2 being displaced with respect to the spacer 3 in the direction indicated by arrow 30, the curved insertion part 4 will, as it were, move out of the follower part 5 of the basic element 2 and will move towards the rear side 45 of the pharynx 40. The insertion part 4 can come into contact with the rear side 45 of the pharynx 40 and come to a standstill there, but it is also possible for the insertion part 4 to remain at a distance from the rear side 45 of the pharynx 40. If, from this situation, the basic element 2 is then moved further in the direction indicated by arrow 30, space is created in the pharynx 40 as a result of the insertion part 4 and the follower part 5 moving away from one another and the follower part 5 in the process pushing away the tongue 41 in the direction of the row of teeth 43. Moreover, as a result of the insertion part 4 and the follower part 5 being moved away from one another, the epiglottis 44 will fold over towards the tongue 41, as indicated by arrow 17. In this context, it is advantageous for the end 12 of the follower part 5 to extend beyond the insertion end 11 of the insertion part 4 (cf. Figure 3). As a result, when the instrument 1 is introduced into the pharynx 40, the end 12 will move into the angle between the epiglottis 44 and the base of the tongue, without the insertion end 11 coming close to the epiglottis 44. As a result, the epiglottis 44 will fold over towards the tongue 41 without being impeded, and it is possible to prevent damage to the epiglottis.

As a result, the opening of the trachea 46 is completely or at least partially uncovered, or at any rate the epiglottis 44 is made to close off the opening to the trachea 46 to a lesser extent. Moreover, the opening to the trachea 46 is opened further as a result of the spreading of the pharynx 40, i.e. the larynx is pushed away from the rear side 45 of the pharynx 40, so that space is created in the pharynx 40. When the instrument 1 is in the holding-open position shown in Figure 4, it is then possible for an artificial respiration mask, which is connected to an artificial respiration apparatus, to be placed over the mouth of the person in order to assist breathing. This is not shown in more detail in the figure.

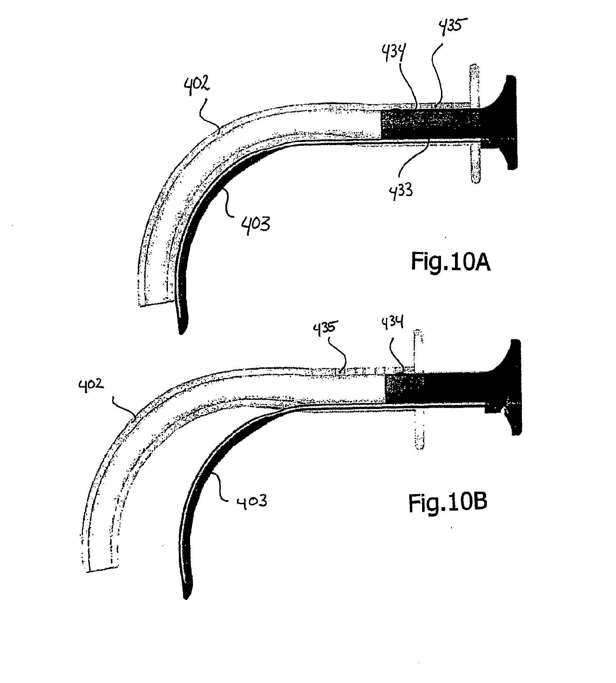

To keep the instrument 1 in the holding-open position shown in Figure 4, it is advantageous for the instrument 1 to be provided with suitable locking means which ensure that the basic element 2 and the spacer 3, in the open position of the instrument 1, can no longer move with respect to one another. This can be achieved, for example, by positioning a clip over the first and second delimiting bodies 14, 31. A clip of this type will press the delimiting bodies 14, 31 onto one another and will prevent the basic element 2 from sliding back outwards under the influence of compressive forces exerted thereon. Other possible options are also conceivable. By way of example, one example is described with reference to Figures 10A-10C.

If the basic element 2 is made from a flexible material, the upper part of the insertion part 4, if it is supported against the rear side 45 of the pharynx 40, will seek to move further if the person handling the instrument 1 continues to exert force in the direction of arrow 30, and a displacement of the upper part of the insertion part 4 will lead to an increase in the curvature thereof, and the angle and/or direction in which the insertion end 11 of the insertion part 4 is oriented with respect to the opening of the trachea 46 will change. As a result of the insertion part 4 being moved further into the pharynx 40 from this position, the curvature of the insertion part 4 will change as a result of the counterpressure which the rear wall 45 of the pharynx 40 exerts on the insertion part 4.

The degree of curvature of the insertion part 4 should preferably be such that the insertion end 11 of the insertion part 4 is directed to a point located beneath the epiglottis 44. This is because the epiglottis 44 marks the start of the trachea 46, and as a result of the insertion end 11 being directed in this way the air stream which is to be passed through the instrument 1 will enter the trachea 46 more easily.

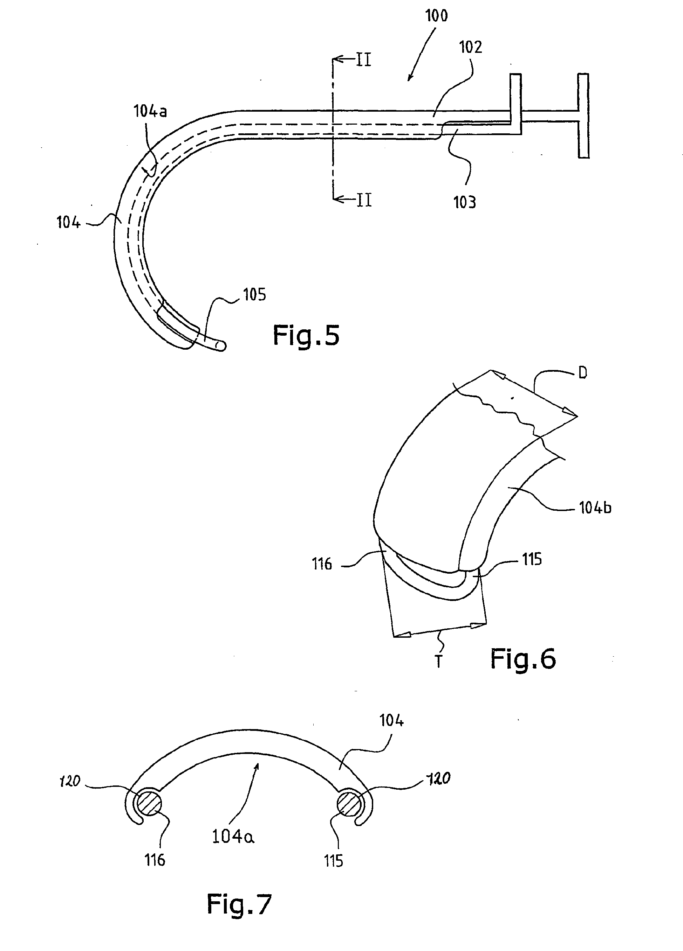

Figure 5 shows an alternative embodiment of the instrument according to the invention in a side view, this instrument being denoted overall by reference numeral 100. The instrument 100 likewise comprises a basic element 102, which is substantially identical to the basic element 2 as shown in the previous figures and therefore likewise comprises a curved insertion part 104. Furthermore, the instrument 100 comprises a substantially J-shaped spacer 103 with a curved follower part 105. Instead of a form-fitting through-flow passage, the basic element 102 is formed as a channel which extends in the longitudinal direction and is delimited by upright edges. The basic element 102 has an inner surface 104a.

The follower part 105 has a larger dimension D in the width direction than the space T between the limbs 115 and 116 of the spacer 103. This is shown in Figure 6. This means that the inner surface 104a of the insertion part 104 rests on the follower part 105 when the instrument 100 is in the introduction position. For this purpose, the insertion part 104 comprises a longitudinal edge 104b which is of elevated design and drops over the follower part 105. This is illustrated in Figure 5 by a dotted line. In the raised longitudinal edge 104b, the insertion part 104 preferably comprises a recess 120 in which the limbs 115 and 116 of the follower part 105 can be accommodated. This is diagrammatically indicated in Figure 7, which shows a cross section through the instrument 100 on line II-II in Figure 5.

The curvature of the insertion part 104 is matched to the curvature of the follower part 105, in order to create an instrument which is as compact as possible. However, the curvature of the follower part 105 is selected in such a way that this curvature will be able to follow the contour of the tongue as successfully as possible. However, this is not necessary; what is primarily important is that the tongue is ultimately successfully confined against the follower part 105. The insertion part 104 may comprise a substantially flat cross section, although it is also possible for the insertion part 104 to comprise a more round cross section, as shown in Figure 7.

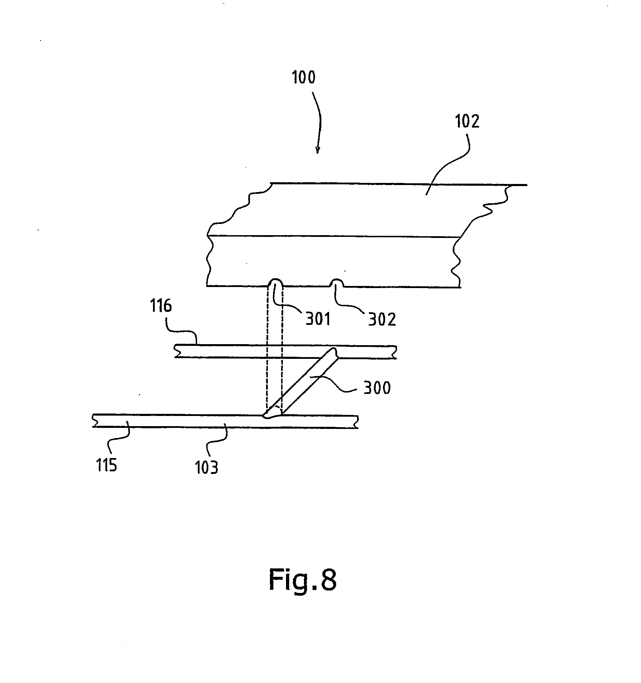

The use of the instrument 100 according to the present invention can advantageously be simplified still further by providing the instrument 100 with locking means which make it possible to secure the instrument 100 which has been introduced into the pharynx 40 in the holding-open position. One possible option is shown in Figure 8.

Figure 8 shows a diagrammatic, cut-away, perspective illustration of part of the instrument 100. The spacer 103 comprises an intermediate piece 300 which is arranged between the limbs 115, 116 and can be accommodated in receiving openings 301, 302, which are provided for this purpose in the basic element 102, as indicated by dashed lines in the figure.

As a result of the basic element 102 being positioned with the receiving openings 301, 302 above the intermediate piece 300, displacement of the basic element 102 with respect to the spacer 103 is locked, so that the person handling the instrument 100 no longer has to exert any force and the instrument 100 can rest in the holding-open position in the pharynx 40.

In the embodiment shown in Figure 8, the basic element 102 comprises four receiving openings 301, 302, but only two of these are visible, and the spacer 103 comprises one intermediate piece 300. However, it will be clear that a different number of receiving openings and/or intermediate pieces are also possible.

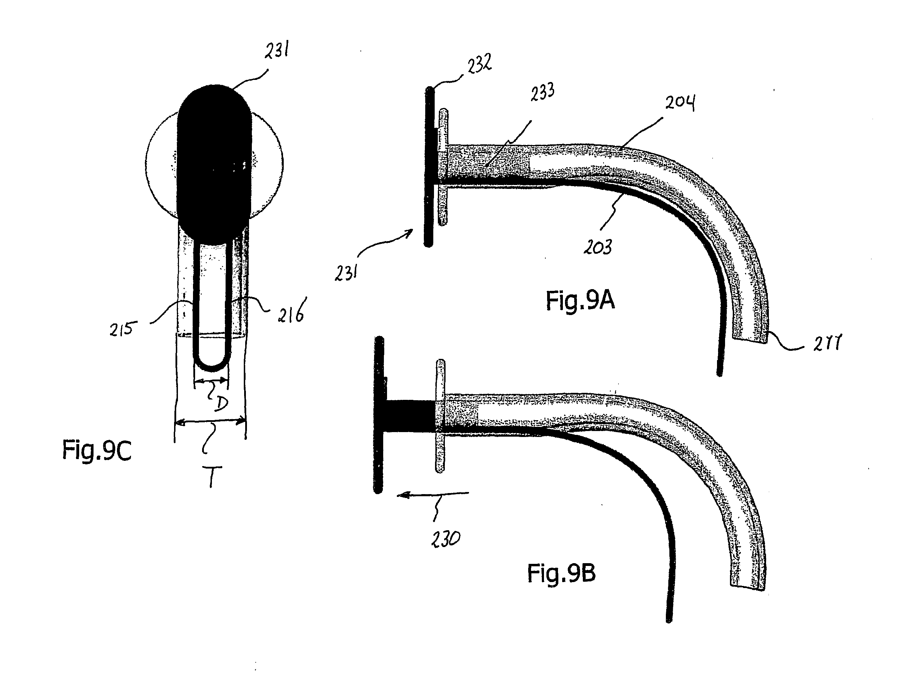

An alternative embodiment of the instrument according to the invention is shown in Figures 9A, 9B and 9C.

The above text describes the instrument according to the invention, and in particular its use, working on the basis that the instrument is introduced into the pharynx and is then moved from the introduction position into the holding-open position as a result of the basic element being displaced along the spacer by pushing. In this case, the straight section of the basic element extends further than the straight section of the spacer.

However, it is also possible to make the straight section of the spacer longer than the straight section of the basic element, so that the displacement of the basic element and the spacer with respect to one another is achieved by introducing the instrument into the pharynx and then displacing the spacer along the basic element by means of a pulling movement so that it is, as it were, pulled out of the pharynx. This is shown in Figures 9A-9C.

Figure 9A shows the instrument in its introduction position, Figure 9A showing the introduction position and Figure 9B showing the holding-open position. The instrument once again comprises the basic element with the insertion part 204 and curved insertion end 211. The spacer 203 is formed as a metal wire which has been bent into a J shape, the limbs 215, 216 having a width D which is less than the width T of the basic element (cf. Figure 9C). At the free end of the straight part, the spacer 203 is coupled to a delimiting body 231. The delimiting body 231 comprises a closure plate 232. The basic element in turn comprises a delimiting body.

It can be clearly seen that the delimiting bodies bear against one another in the introduction position instead of being at a distance from one another as shown, for example, in Figure 3.

The spacer 203 projects partially into the delimiting body 231. The spacer 203 furthermore comprises a coupling part 233. The coupling part 233 extends partially into the basic element and can be displaced therein. This is shown in Figure 9B, in which the spacer 203 has been pushed out of the basic element 204, in the direction indicated by arrow 230, into the holding-open position of the instrument. Figures 9A and 9B clearly show that the spacer 203 projects partially through the wall of the basic element and is supported in such a manner that it is guided therein.

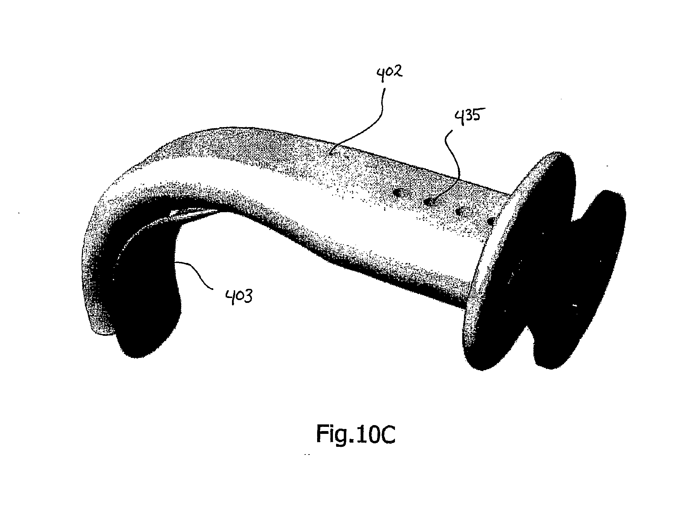

Figures 10A, 10B and 10C show a variant of the instrument shown in figures 9A-9C.

Figure 10A shows, in the introduction position thereof, substantially the same instrument as that shown in Figures 9A-9C, but in this case the spacer 403 is designed as a closed, spatula-like component instead of a component with an open structure. Furthermore, the instrument comprises locking means in the form of a coupling projection 434 which is arranged on the coupling part 433 and can engage in corresponding receiving openings 435 which are arranged in the wall of the basic element 402. In the case shown in Figures 10A-10C, the basic element 402 and the spacer 403 can adopt four different positions with respect to one another. The position of the receiving openings 435 is clearly visible in Figure 10C. The coupling projection 434 produces a click-fit connection, which is made possible by the fact that the material of the basic element 402 or the coupling element 433 or both is flexibly deformable, to which end the basic element 402 and/or the coupling part 433 may advantageously be made from plastics.

In the above text, the invention has been discussed on the basis of an instrument for creating space in the pharynx of a person, the instrument being designed to carry artificial respiration air from the mouth to the trachea.

A second aspect of the inventive idea is that by creating space in the pharynx it becomes possible to use an instrument according to the invention to introduce an artificial respiration tube into the trachea of a person without the entry to the trachea having to be visible to the person handling the instrument. This will be explained in more detail in the following.

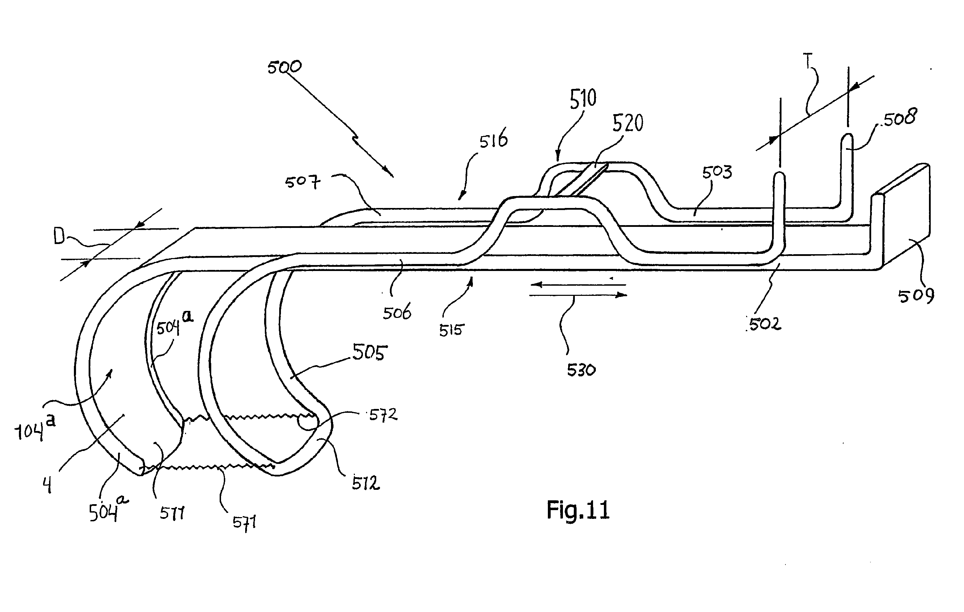

Figure 11 shows an instrument according to the invention which is denoted overall by reference numeral 500. The instrument 500 comprises a spacer 503 and a basic element 502. The spacer 503 and the basic element 502 are both substantially J-shaped. The spacer 503 comprises a curved follower part 505 having a free end 512, which follower part 505 is designed to follow the contour of a tongue. The basic element 502 comprises a curved insertion part 504 having an insertion end 511. Furthermore, the spacer 503 and the basic element 502 each comprise a substantially straight section 507 and 506, respectively, which adjoins the follower part 505 and the insertion part 504, respectively.

As shown in the figure, the spacer 503 has an open structure and is preferably formed as a substantially J-shaped framework, for example made from a tubular material, and comprises two substantially parallel limbs 515 and 516. A space which is denoted by T is defined between the limbs 515 and 516 of the spacer 503. The basic element 502 is formed as a substantially J-shaped strip of material which has a substantially channel-shaped cross section, the dimension of the basic element 502 at right angles to the longitudinal direction in the figure being denoted by D. The dimension D of the basic element 502 is smaller than the space T and therefore the basic element 502 fits into the space T which the framework of the spacer 503 defines, and it is possible for the basic element 502 to move through the spacer 503.

The spacer 503 has a free end 508, of which the corresponding ends of the spacer 503 are bent upwards in a direction substantially perpendicular to the plane thereof. The basic element 502 has a free end 509 which comprises an end which is directed upwards substantially at right angles. The free ends 508 and 509 in this way form gripping means for handling the instrument 500.

The substantially straight section 507 of the spacer 503 is provided, between the free end 508 and the follower part 505, with a substantially parallel raised section 510 which is located at a higher level than the plane of the straight section 507. Between the two limbs 515, 516 of the spacer 503 there is a transverse connection 520 which spans the space T. The transverse connection 520 functions as an additional reinforcement for the spacer 503.

In the configuration shown in the figure, the basic element 502 is located in the open structure or space T of the spacer 503 and therefore projects through the spacer 503. Since the basic element 502 is located between the limbs 515, 516 of the spacer 503, lateral displacement of the basic element 502 with respect to the spacer 503 is prevented. In particular, the elevated section 510 and to a lesser extent the raised ends of the free end 508 of the spacer 503 are responsible for limiting lateral movement of the basic element 502, but do allow displacement substantially in the longitudinal direction. This is indicated by a double arrow 530 in the figure.

However, the spacer 503 and the basic element 502 cannot be displaced to an unlimited extent in the longitudinal direction with respect to one another. The follower part 505 and the insertion part 504 are coupled to one another in the vicinity of their ends 512 and 511 by means of coupling means 571, 572. The coupling means 571, 572 are preferably formed as flexible cord-like bodies. These are secured to the outwardly directed sides of the limbs 515, 516 of the spacer 503 and to the outwardly directed longitudinal edges 504a of the basic element 502. As a result, displacement of the insertion part 504 through the follower part 505 of the spacer 503 will not be impeded and, moreover, the space T only has to be slightly larger than the width D of the basic element 502. This results in the minimum possible play in the transverse direction between the spacer 503 and the basic element 502.

The coupling means 571, 572 are preferably formed from a flexible but inelastic wire, though it is also possible to use an elastic wire. However, it is also possible for the coupling means 571, 572 to be of substantially rigid design. In this case, it is important for rigid coupling means to be pivotably coupled to the spacer 503 and the basic element 502 at their ends. It is also possible for the insertion part 504 and the follower part 505 to be coupled to one another using substantially rigid coupling means which can move with respect to one another. However, it is preferable for the coupling means 571, 572 to be formed by a flexible wire in order to prevent the risk of internal injury. It is important for the coupling means 571, 572 to have a limited length, so that the basic element 502 can only be moved a limited distance with respect to the spacer 503, and for it to be possible for the insertion part 504 to be deformed, as will be explained in more detail in the text which follows. In this context, it will be clear that the length of the coupling means 571, 572 can be made dependent on the question of whether a child or an adult is being treated with the instrument.

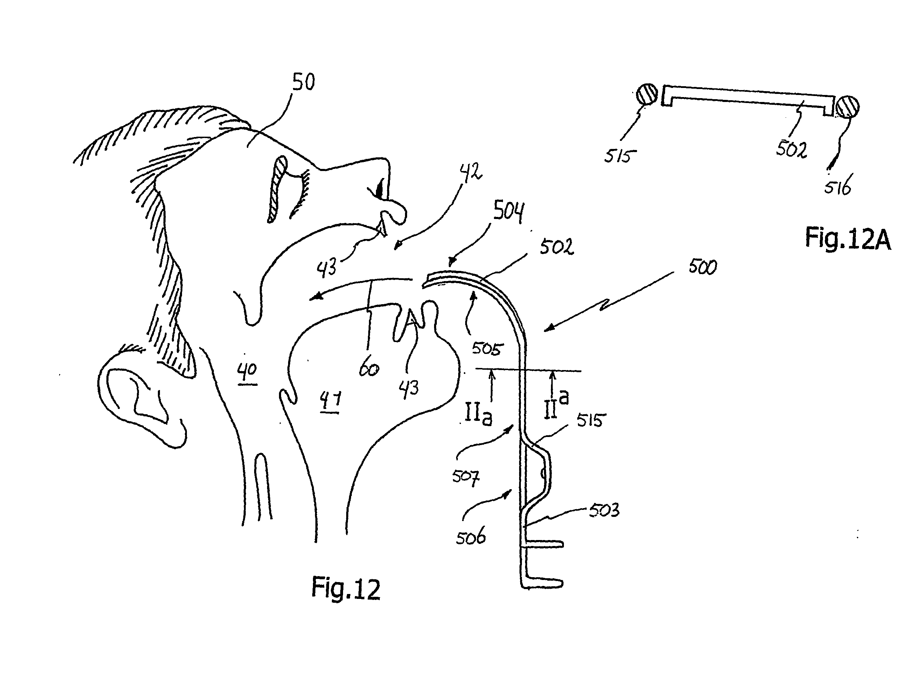

A use of the instrument according to the invention will be explained in more detail with reference to the following description of Figures 12-15.

Figure 12 shows the instrument 500 in the introduction position just outside the mouth opening 42 of a person who is in a lying position, only part of a head 50 of the person having been illustrated in order to simplify the drawing. The head 50 contains the tongue 41, the pharynx 40 and the row of teeth 43. In the introduction position shown, the instrument 500 is in a closed position, which in this case means that the basic element 502 lies completely or mainly between the limbs 515, 516, of which only the limb 515 is visible in the figure. Moreover, the insertion part 504 virtually coincides with the follower part 505. In this way, the combined thickness of the follower part 505 and the insertion part 504 and the straight sections 507, 506 of the spacer 503 and the basic element 502 which directly adjoins them is substantially the same as the maximum thickness of one of the two elements. As a result, the thickness of the instrument 500 at the location of its section which has to move past the row of teeth 43 is very small, which is highly advantageous if the instrument 500 has to be used for people who cannot open their mouth opening 42 sufficiently far. To illustrate the small thickness, Figure 12A shows a cross section on line IIa-IIa in Figure 12.

From the position shown, the instrument 500 can be introduced into the open mouth 42 of the person in a direction which is indicated by arrow 60. This is shown in Figure 13.

Figure 13 shows the instrument 500 in a position in which the instrument is still in the introduction position but in which at least the follower part 505 and the insertion part 504 are inside the pharynx 40 of the person. In order for the instrument 500 to be moved into the position shown in the figure, the instrument 500 has been introduced by moving the spacer 503 with the follower part 505 past the row of teeth 43 and then guiding the follower part 505 along the surface of the tongue 41 into the pharynx 40 until the free end 512 of the follower part 505 rests in an angle which is enclosed between the tongue 41 and the epiglottis 44. In this way, the introduction of the instrument 500 reaches a natural end and it is possible to prevent the instrument 500 from being pushed too far into the pharynx 40.

As can be clearly seen in the figure, the follower part 505 hooks into the angle between the epiglottis 44 and the tongue 41 in such a manner that the instrument 500 cannot easily be removed from the pharynx 40. The only way in which the instrument 500 can be removed again from the pharynx 40 is by the follower part 505 being guided back over the surface of the tongue 41 by means of a tilting movement (in a clockwise direction in the figure). This ensures reliable positioning of the instrument 500.

To prevent the free end 512 of the follower part 505 from coming out of the said angle and/or to further simplify positioning of the instrument 500 in the pharynx 40, it is advantageous to provide the free end 512 of the follower part 505 with a recess 561. This is diagrammatically illustrated in Figure 14, which shows only that section of the spacer 503 or the follower part 505 which comprises its free end 512. The recess 561 is preferably rounded in order to prevent injury to the tongue 41 and/or the epiglottis 44. However, the recess 60 may also be of another suitable shape.

In addition, it is expedient to allow the follower part 505 to extend beyond the insertion part 504, so that the end 512 is situated below the insertion end 511. The advantages of this have already been discussed with reference to Figure 4 and also apply to the instrument which is currently under discussion.

In the situation shown in Figure 13, the person has completely opened his mouth 42. However, as is clearly apparent from the figure, there is still considerable space between the upper teeth and the instrument 500 and it is not necessary for the person to have completely opened his mouth 42 in order to enable the instrument 500 to be introduced. On account of the small thickness of the instrument 500, in particular that section which has to move past the row of teeth 43, the person may even have his mouth 42 almost closed without this making it difficult to introduce the instrument 500. Since the contour of the tongue 41 is followed during the introduction of the instrument 500, the free end 512 of the spacer 503 always reaches the intended location in the pharynx 40, namely the angle which is enclosed by the tongue 41 and the epiglottis 44.

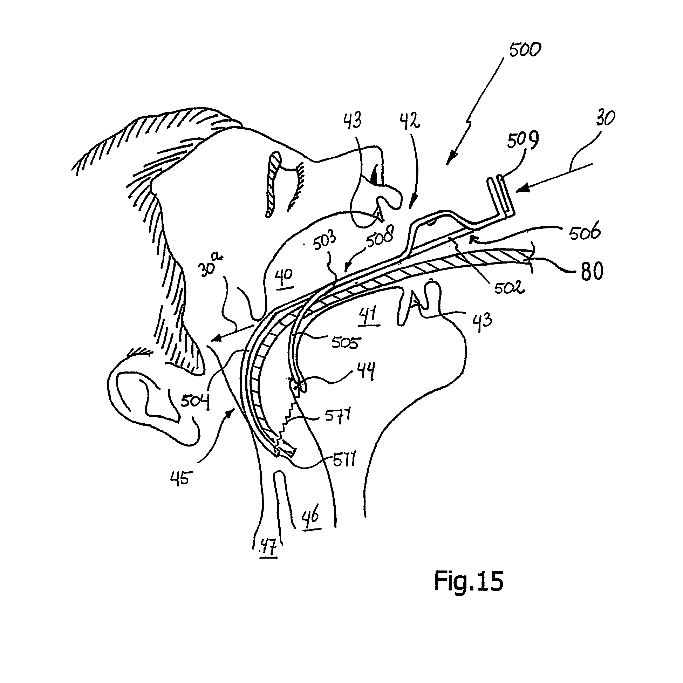

The following step of introducing the instrument 500 into the pharynx 40 is explained in more detail with reference to Figure 15.

Figure 15 shows the instrument 500 in the holding-open position. From the introduction position shown in Figure 13, the person handling the instrument 500 displaces the basic element 502 in a substantially longitudinal direction with respect to the spacer 503, as indicated in the figure by arrow 30. The person handling the instrument 500 can use the grip means for this purpose.

As a result of the displacement of the basic element 502 in the direction of arrow 30, the curved insertion part 504 thereof will, as it were, move out of the curved follower part 505 of the spacer 503 and move towards the rear side 45 of the pharynx 40. The insertion part 505 can come to a standstill against the rear side 45 of the pharynx 40, but may also remain at a (short) distance therefrom. If, from this situation, the basic element 502 is then moved further in the direction of arrow 30, in the first instance space will be created in the pharynx 40 as a result of firstly the insertion part 504 and the follower part 505 moving away from one another and as a result of the follower part 505 in the process moving the tongue 41 in the direction of the row of teeth 43. In addition, as a result of the insertion part 504 and the follower part 505 being moved away from one another, the epiglottis 44 will fold over in the direction of the tongue 41, so that the opening of the trachea 46 is completely uncovered. In this case, therefore, the opening of the trachea 46 is opened up as a result of the spreading of the pharynx 40, i.e. the larynx is pushed away from the neck vertebrae (rear side 45), so that space is created in the pharynx 40. In the second instance, the movement of the insertion part 504 and the follower part 505 away from one another will come to an end as a result of the restricted length of the coupling means 571, 572. However, if the person who is using the instrument 500 continues to exert force on the basic element 502 in the direction of the arrow 30, the upper part of the insertion part 504 will seek to move further, as indicated by arrow 30A. Therefore, a movement of the upper part of the insertion part 504 will lead to an increase in its curvature, and the angle and/or direction at which the insertion end 511 of the insertion part 504 is directed with respect to the opening of the trachea 46 will change. Even if the outwardly directed surface of the curved insertion part 504 is supported against the rear side 45 of the pharynx 40 before the coupling means 571, 572 have been completely extended, the upper part of the insertion part 504 will seek to move further, which will lead to an increase in its curvature. As a result of the insertion part 504 being moved further into the pharynx from this position, so that the insertion part 504 comes into contact with the rear side 45 of the pharynx 40, the counterpressure exerted by the rear wall 45 of the pharynx on the insertion part 504 will cause the curvature of the insertion part 504 to change.

To make it easy for the change in the curvature of the insertion part 504 to take place, at least this curved insertion part 504 is made from an elastically deformable material, in particular an elastically deformable plastic material. This plastic material should preferably be suitable for medical applications. To further simplify the deformation of the insertion part 504, the insertion part 504 can be of weakened design. It is preferable for the straight section 506 of the basic element 502 to be provided with vertical edges, so that the force which is exerted on the free end 509 will only effect a deformation of the curved insertion part 504 and not of the straight section, or at least to a much lesser extent. Moreover, for the same reason the spacer 503 is of relatively rigid design. This makes it possible to prevent the straight sections 506 and 507 from being able to buckle, in the process opening the mouth of the person and exerting forces on the row of teeth 43.

Then, a tubular object 80, in particular an artificial respiration tube, can be introduced into the pharynx 40. For this purpose, the tubular object 80 is passed from outside the mouth 42 through the open structure of the spacer 503 and via an inner surface 104a (cf. Figure 1) of the basic element 502 into the pharynx 40.

The degree of curvature of the insertion part 504 should preferably be such that the insertion end 511 of the insertion part 504 is directed to a point which lies below the epiglottis 44 or the free end 512 of the follower part 505. This is because the epiglottis 44 marks the beginning of the trachea 46 and, as a result of the insertion end 511 being directed towards the said point, it will be possible for the tubular object 80 to be successfully introduced into the trachea 46.

In order for the tubular object 80 to be guided successfully along the inner surface 104a, the invention provides at least a section of the basic element 502 with delimiting members which extend in the longitudinal direction in order to form a guide channel. Delimiting members of this type may, for example, be designed as an elevated longitudinal edge or as a U-shaped or ∩-shaped channel arranged on the inner surface 104a. It will be clear that numerous different variants are possible.

It can be seen from Figure 15 that the insertion part 504 largely closes off the opening of the oesophagus 47. For the invention, it is advantageous for the curvature of the insertion part 504 to extend sufficiently far in the direction of the trachea 46 or the oesophagus 47, i.e. for the insertion part 504 to comprise sufficient length, to prevent damage to the rear side 45 of the pharynx 40 during the introduction of the instrument 500. If the length of the insertion part 504 is too short, its insertion end 511 will scrape along the rear side 45 of the pharynx 40, with the risk of injury. It is therefore possible for the insertion part 504 to have a sufficient length to prevent injury but insufficient length to close off the opening of the oesophagus 47. However, this is not necessary since the angle between the insertion part 504 and the trachea 46 can be changed in such a way that the tubular object 80 which is to be introduced with the aid of the instrument 500 does not unintentionally pass into the oesophagus 47.

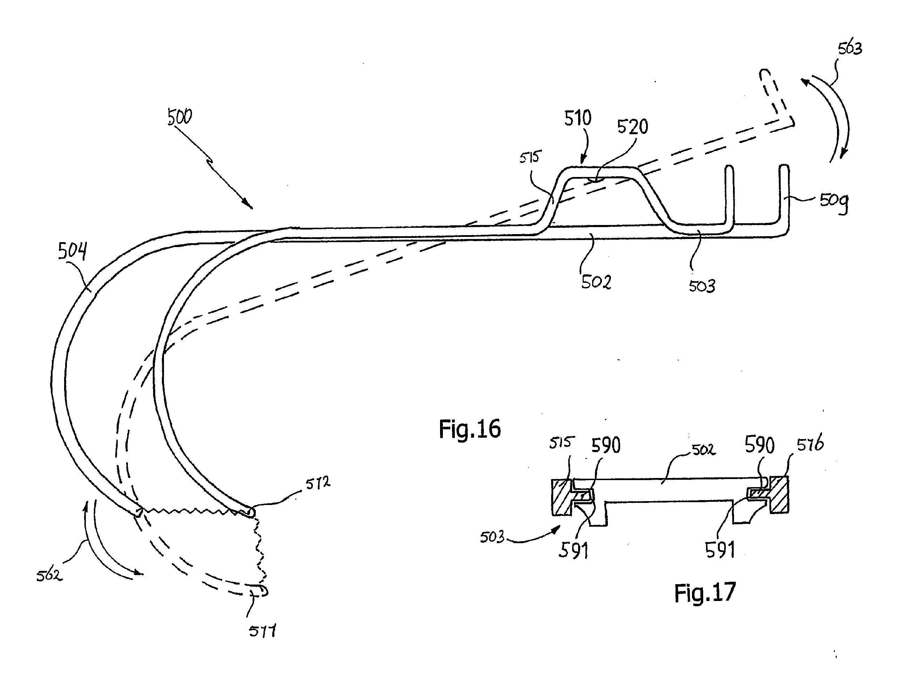

An alternative possibility for changing the angle and/or direction at which the insertion end 511 is oriented towards the opening of the trachea 46 is offered by interaction between the straight section 506 of the basic element 502 and the transverse connection 520 which functions as a support member. This is explained in more detail below with reference to Figure 16. As has already been discussed in connection with the explanation of Figure 11, the transverse connection 520 is arranged between the two limbs 515, 516 of the spacer 503. The transverse connection 520 also functions as a support member for the basic element 502. In particular, the transverse connection 520 makes it possible for the basic element 502, which in the configuration shown in Figure 16 can rest freely against the transverse connection 520, to tilt or pivot about the transverse connection 520. As a result of the basic element 502 being pressed against the transverse connection 520 and then the basic element 502 being moved upwards or downwards slightly via its free end 509, in a direction as indicated by a double arrow 563, the transverse connection 520 will function as a supporting and tilting point for the basic element 502. As a result of the displacement of the basic element 502 and in particular the free end 509 thereof in one of the directions of the double arrow 563, the insertion end 511 of the insertion part 504 will be moved in the opposite direction. This is shown in the figure by a double arrow 562. A new position which the basic element 502 can adopt is shown in the figure by dashed lines. It should be noted that with a view to simplicity of the illustration, only one new position of the basic element 502 is shown, after an upwards displacement of the free end 509. In this way, the basic element 502 can execute a tilting movement with respect to the spacer 503, the degree of pivoting being limited by the limited length of the coupling means 571, 572. The result of this is that the insertion end 511 can execute a movement with respect to the free end 512 of the spacer 503, in a direction which is substantially at right angles to the longitudinal direction (of displacement). This, therefore, offers a further possibility for changing the angle and/or direction at which the insertion end 511 is directed towards the opening of the trachea 46. It is possible to carry out the introduction of, for example, an artificial respiration tube during the time when the instrument 500 is in the position illustrated in Figure 15 and for the tilting movement described above only to be used thereafter.

An alternative way of creating the possibility of tilting is shown in Figure 17.

Figure 17 diagrammatically depicts a cross section through part of the instrument 500, the limb 515 and the limb 516 of the spacer 503 comprising a cylindrical projection 590 which is directed towards the basic element 502, these projections interacting with guide grooves 591 provided on the basic element 502. As a result, the basic element 502 can slide along the spacer 503 and can also pivot about the cylindrical projections 591. In this case, it is advantageous if the cylindrical projections 591 are arranged at the start of the elevated section 510, as seen from the follower part 505.

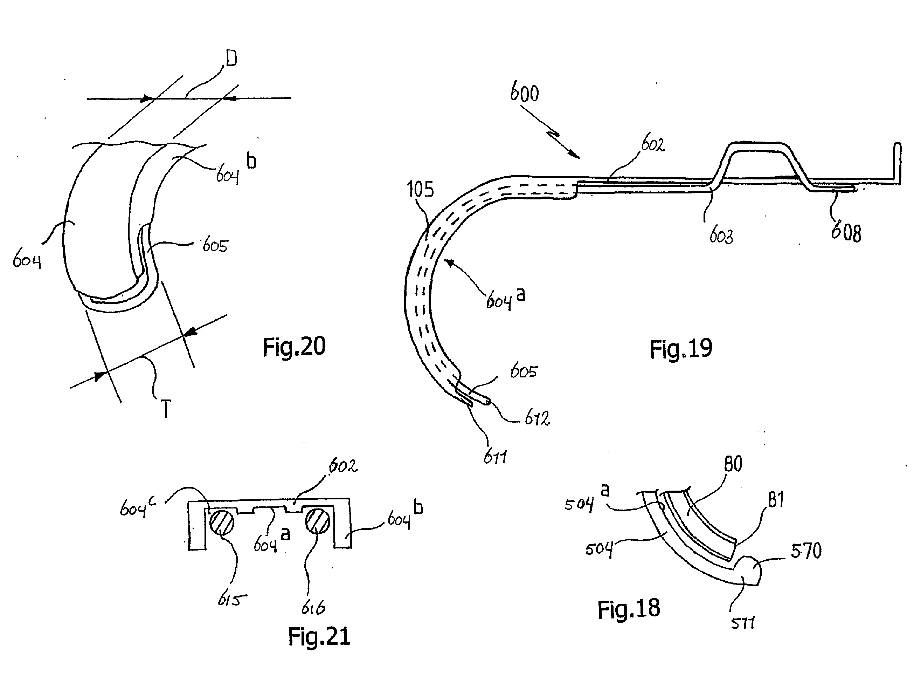

As has already been stated, with the instrument 500 according to the invention it is possible to introduce a tubular object into the trachea 46 of a person. However, an artificial respiration tube of this type is relatively rigid and in practice may not be able to exactly follow the curvature of the curved insertion part 504 of the basic element 502, but rather will have a lesser curvature. In order, in such a case, still to allow the artificial respiration tube to curve sufficiently, it is advantageous for the insertion end 511 of the basic element 502 to be provided with a slight elevation on the inner surface 104a. This is shown in Figure 18.

Figure 18 diagrammatically depicts a longitudinal section through the insertion end 511 of the insertion part 504. At the insertion end 511, there is an elevation 570 which functions as a barrier for an artificial respiration tube 80, which is diagrammatically illustrated in longitudinal cross-section. The end 81 of the artificial respiration tube 80 will come into contact with the elevation 570 when the artificial respiration tube 80 is being introduced along the inner surface 104a of the basic element 502. The elevation 570 prevents the artificial respiration tube 80 from sliding further along the basic element 502, and if the person who is handling the instrument 500 continues to introduce the artificial respiration tube 80, the curvature will increase accordingly. When the curvature of the artificial respiration tube 80 is virtually equal to the curvature of the basic element 502, the end 81 of the artificial respiration tube 80 will jump over the elevation 570 and continue on its way to the trachea 46.

Figure 19 shows a side view of an alternative embodiment of the instrument 500 according to the invention, which is denoted overall by reference numeral 600. The instrument 600 likewise comprises a spacer 603, which is substantially identical to the spacer 503 as shown in the previous figures and therefore likewise comprises a curved follower part 605. Furthermore, the instrument 600 comprises a substantially J-shaped basic element 602 with a curved insertion part 604. Unlike the embodiment of the instrument 600 which has been shown in the previous figures, the coupling means are absent and the insertion part 604 has a larger dimension D in the width direction than there is space between the limbs 615 and 616 of the spacer 603. As a result, it is possible, after the instrument 600 has been used to introduce an object 80, for the basic element 602 and the spacer 603 to be removed from the pharynx 40 separately. This is shown in Figure 19.

This means that the inner surface 604a of the insertion part 604 rests on the follower part 605 when the instrument 600 is in the introduction position. For this purpose, the insertion part 604 comprises a longitudinal edge 604b which is of elevated design and engages over the follower part 605. This is shown by a dashed line in Figure 19. Preferably, the insertion part 604 comprises a recess 604c in the elevated longitudinal edge 604b, in which the limbs 615 and 616 of the follower part 605 can be received. This is diagrammatically depicted in Figure 21.

The curvature of the insertion part 604 is matched to the curvature of the follower part 605, in order to create an instrument which is as compact as possible. However, the curvature of the follower part 605 is selected in such a way that this curvature will be able to follow the contour of the tongue as fully as possible. In some cases, this may result in the curvature of the insertion part 604 being insufficient to pass the entry to the oesophagus and in the tubular object 80 which is to be introduced unintentionally passing into the oesophagus.

In order nevertheless to be able to change the curvature of the insertion part 604 of the instrument 600 shown in Figure 19 in a suitable way, the invention proposes the following.

In the introduction position of the instrument 600 shown in the figure, the inner surface 604a of the insertion part 604 rests on the follower part 605, the follower part 605 and the insertion part 604 having substantially the same radius. However, if the insertion part 604 is provided with a "pre-curvature", i.e. if the insertion part 604, in a stress-free state, has a curvature which is greater than the curvature of the follower part 605, movement of the insertion part 604 and the follower part 605 away from one another will lead to the insertion part 604 adopting its "natural" position, so that it will automatically adopt a more curved position than the follower part 605. Therefore, in an open position of the instrument 600, the insertion part 604 is at a distance from the follower part 605 and, in this position, has a smaller radius than the follower part 605. In this way, it is still possible for the instrument 600 to be provided with the minimum possible thickness in combination with the possibility of adapting the curvature of the insertion part 604 without using coupling means in the vicinity of the ends 612, 611 of the follower part 605 and the insertion part 604.

It should be noted that the instrument according to the invention, which is suitable for the introduction of an artificial respiration tube, is preferably designed in an embodiment in which, after the artificial respiration tube has been introduced, the basic element and the spacer can be removed from the pharynx separately from one another.

The use of the instrument 500 according to the present invention can advantageously be simplified still further by providing the instrument 500 with locking means which make it possible to hold the instrument 500 which has been introduced into the pharynx 40 in the holding-open position, and therefore after that to operate the instrument 500 with one hand. One possible way in which this can be achieved has already been shown above with reference to Figure 8.

It is highly advantageous if the basic element 502 has an open structure. This open structure makes it possible for the spacer to move with a high degree of freedom with respect to the basic element 502 and, moreover, for the tubular object 80 to be introduced easily into the pharynx 40 via the insertion part 503 without the basic element 502 forming an obstacle.

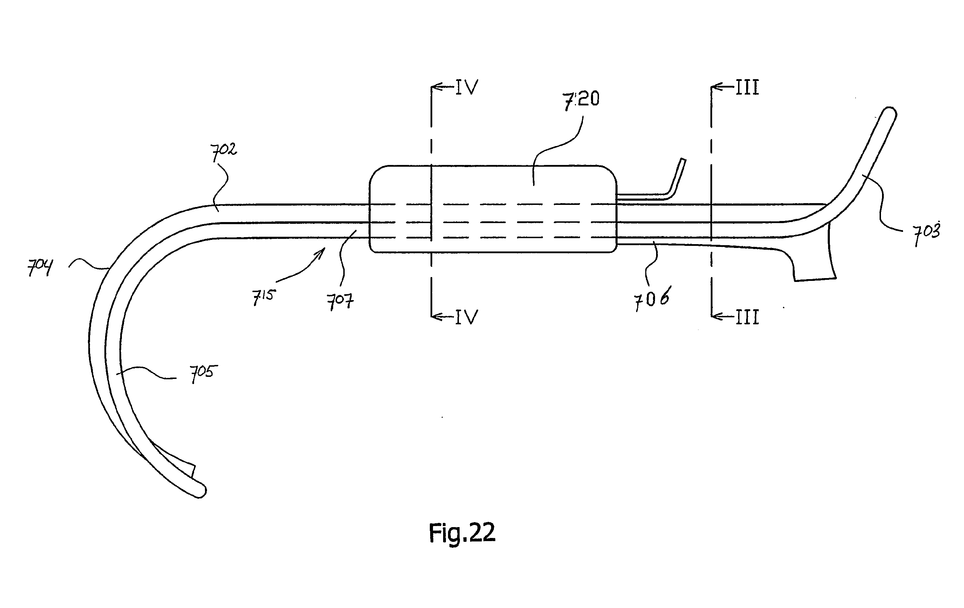

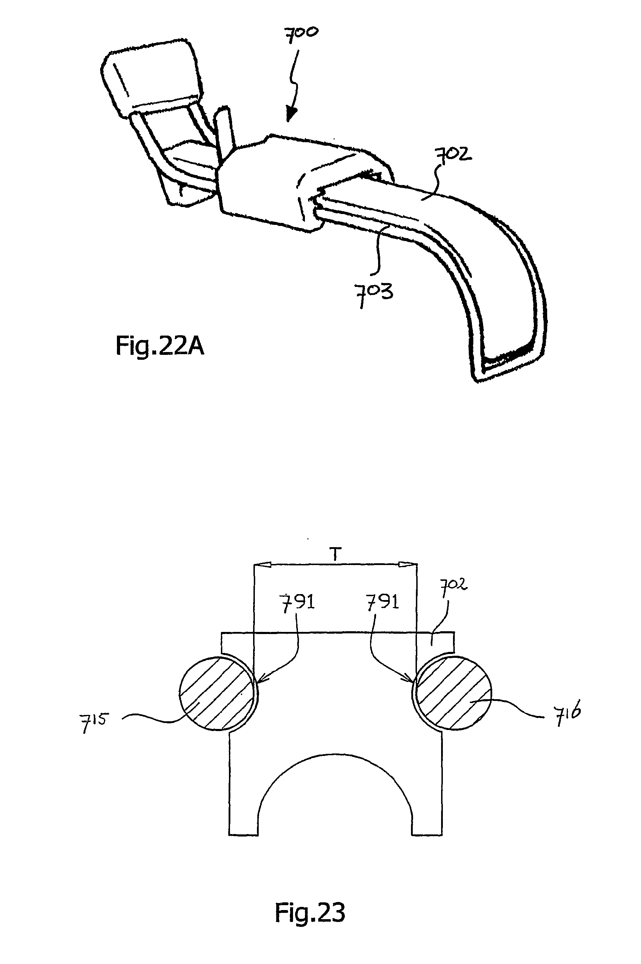

A highly preferred embodiment of the instrument according to the invention is shown in Figure 22.

Figure 22 shows a side view of an instrument 700. The instrument 700 once again comprises a spacer 703 and a curved follower part 705. Furthermore, the instrument 700 comprises a substantially J-shaped basic element 702 with a curved insertion part 704. Unlike in the embodiment of the instrument 500, 600 shown in the previous figures, the straight section 706 does not include an elevated section, but rather is of uninterrupted design. The spacer 703 has a completely open structure, and the support member which forms a transverse connection (cf. Figure 11) is no longer present. This means that the straight section 706 of the basic element 702, in particular at the level of the straight section 707 of the spacer 703, lies completely between the limbs 715, 716. Furthermore, the insertion part 704 has a slightly greater curvature, or in other words a slightly smaller radius, than the follower part 705. Furthermore, the instrument 700 comprises a spreading and locking mechanism 720, by means of which the basic element 702 can be locked with respect to the spacer 703 and by means of which the limbs 715 and 716 can be pushed away from one another in a sideways direction. These aspects will be explained in more detail with reference to the following figures. For the sake of clarity, Figure 22A shows a perspective view of the instrument 700, from which it can clearly be seen that the basic element 702 is located in the open structure of the spacer 703.

Along a section of the straight section 706, the basic element 702 is provided with guide channels which interact with the limbs 715 and 716, so that the basic element 702 can slide along the spacer 703. This is shown in Figure 23.

Figure 23 shows a cross section on line III-III in Figure 22 through the instrument 700. It can be seen from the figure that the basic element 702 is provided at its longitudinal edges with two guide channels 791 which lie opposite one another and in which the limb 715 and the limb 716, respectively, are partially accommodated. The guide channels 791 form a recess in the longitudinal edges of the basic element 702. The limbs 715, 716 of the spacer 703 are sufficiently rigid for the basic element 702 to be clamped between the limbs 715, 716, at least in such a manner that the basic element 702 cannot easily be dislodged from between the limbs 715, 716. In the figure, the limbs 715, 716 are accommodated with play in the guide channels 791, so that the basic element 702 will easily be able to slide along the limbs 715, 716. However, it is also possible for the basic element 702 to be clamped securely between the limbs 715, 716, so that there will be scarcely any lateral play between the basic element 702 and the spacer 703.

However, it is highly advantageous and therefore preferable for it in fact to be possible for the basic element 702 to be removed from between the limbs 715, 716 of the spacer 703, so that the basic element 702 and the spacer 703 can be removed from the pharynx separately from one another. This is the case in particular if the instrument according to the invention is used, for example, to introduce an artificial respiration tube into the trachea, with further components being arranged on the section which remains outside the pharynx of the person, with the result that the part of the artificial respiration tube which lies outside the person cannot easily pass between the space between the follower part and the insertion part if the instrument is to be taken out of the pharynx again.

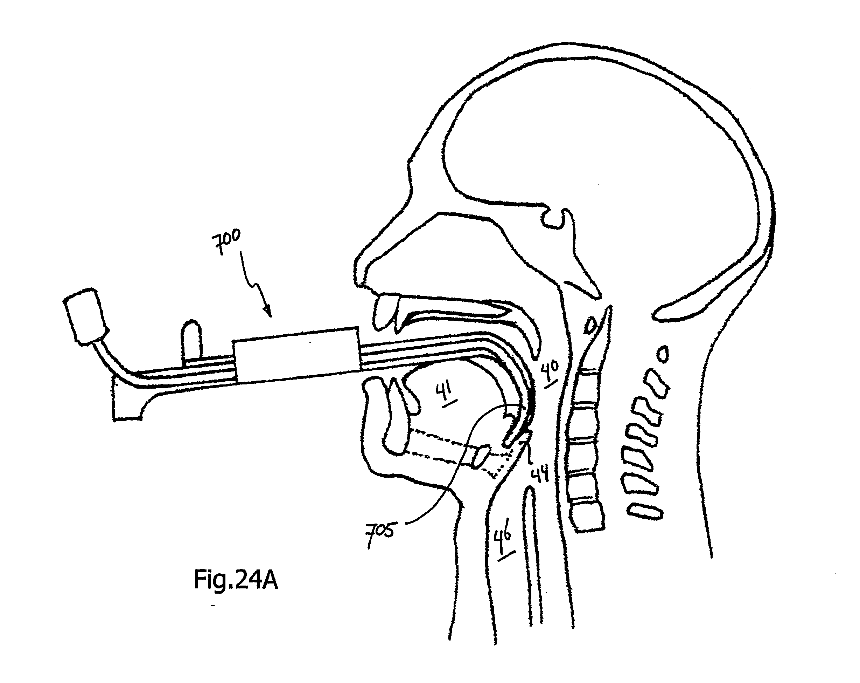

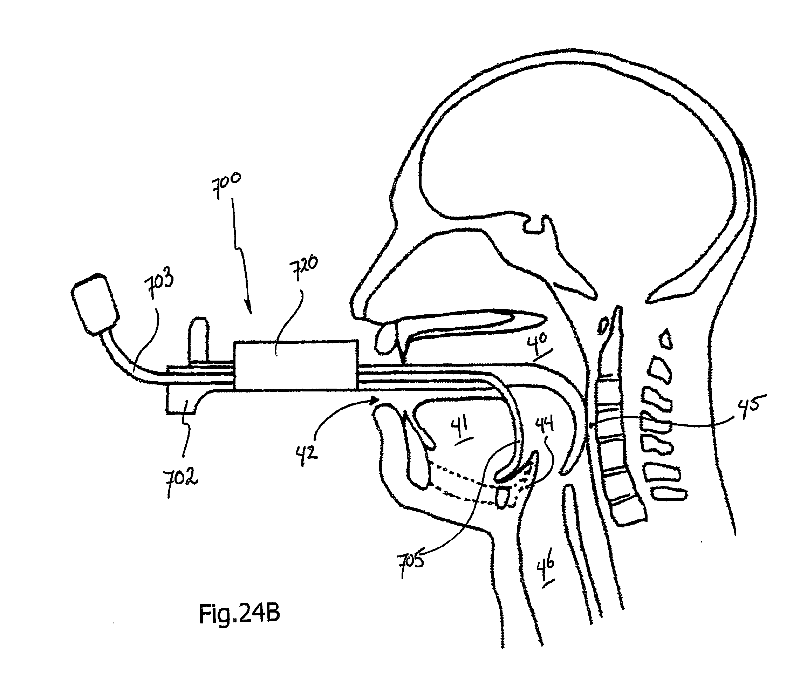

With the instrument 500 shown in Figure 11, this is not possible, inter alia, on account of the coupling between the spacer 503 and the basic element 502 provided by the coupling means 571, 572. With the instrument 600 as shown in Figure 19, this is likewise impossible, firstly on account of the support member 620 and secondly on account of the fact that a part of the basic element 602 has a greater width than the spacer 603. In fact, to allow successful removal of the spacer 703 and the basic element 702 separately from one another from the pharynx, it is necessary firstly to remove the basic element 702 and then the spacer 703, with the basic element 702 being pulled over the artificial respiration tube 80 which has been introduced. Once the basic element 702 has been removed from the pharynx 40, it is easy to remove the spacer 703. This is diagrammatically summarized in Figures 24A-24E.

Figure 24A shows the instrument 700 in a position in which it has been introduced into the pharynx 40. The instrument 700 is still in its introduction position. It can clearly be seen from Figure 24A that the follower part 705 does not necessarily have to precisely adjoin the contour of the tongue 41. It can also be seen that the epiglottis 44 partially closes off access to the trachea 46. From the position shown in Figure 24A, the basic element 702 is displaced with respect to the spacer 703 in the direction of the rear side 45 of the pharynx 40. As a result, the epiglottis 44 folds over in the direction of the tongue 41 and largely clears access to the trachea. It can also be seen that as a result of the displacement of the basic element 702 with respect to the spacer 703, the tongue 41 is pressed towards the mouth opening 42, and that the follower part 705 closely adjoins the contour of the tongue. The instrument 700 can be locked in this holding-open position by means of the spreading and locking mechanism 720. It is preferable for the spreading and locking mechanism 720 to be designed in such a way that, during the displacement of the basic element 702 with respect to the spacer 703, locking is automatically accomplished. This can be achieved, for example, by a click-action mechanism with a number of discrete positions, the click-action mechanism only allowing displacement of the basic element 702 out of the spacer 703 and thereby preventing return displacement. This can be achieved, for example, by a resilient element which is provided in the spreading and locking mechanism 720 and interacts with toothing provided on the basic element 702. This is not shown in more detail in the figures.

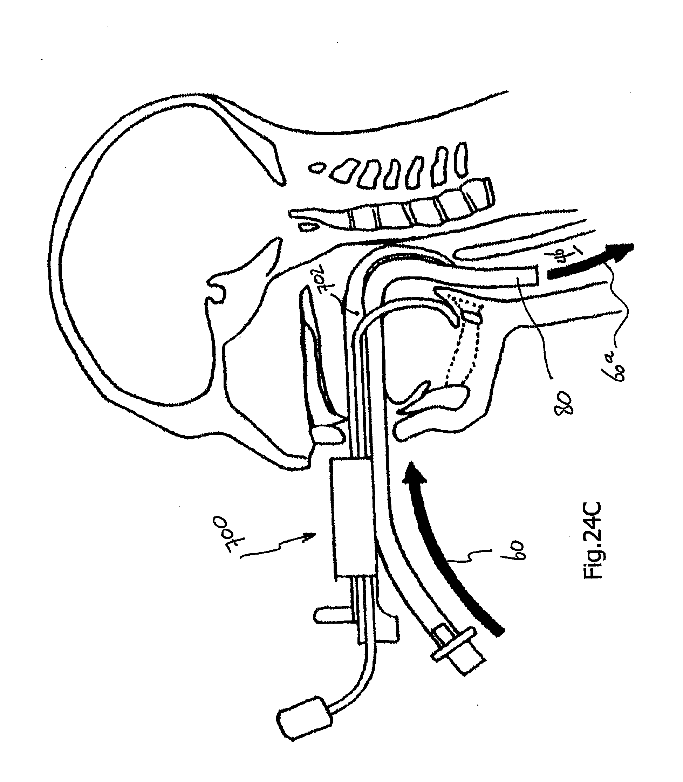

It can be seen from Figure 24C how the artificial respiration tube 80 is introduced into the trachea 46 via the pharynx 40 and via the basic element 702. This is illustrated with the aid of direction arrows 60 and 60a.

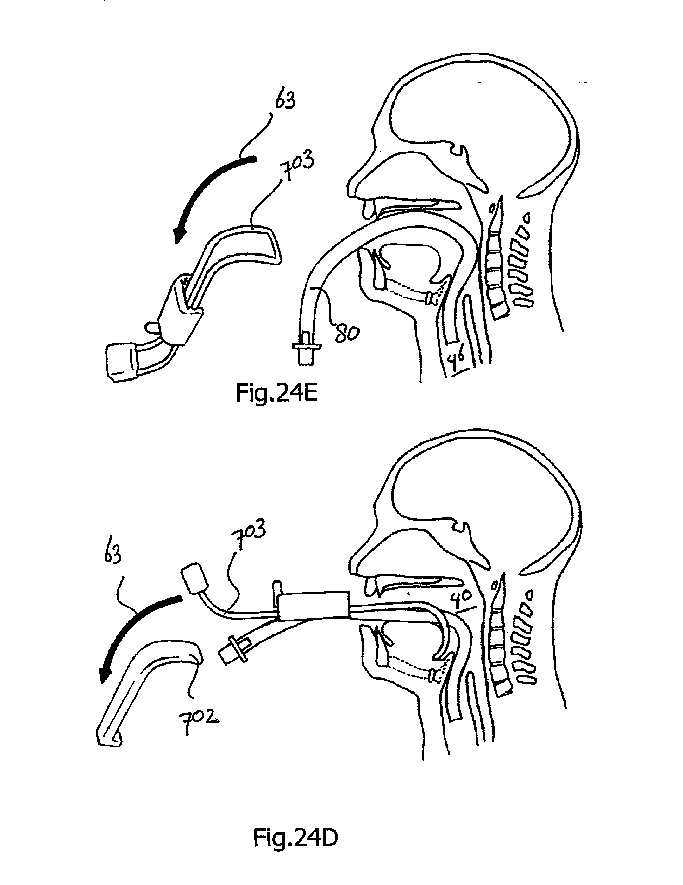

The removal of the instrument 700 is diagrammatically depicted in Figure 24D and 24E. First of all, the locking between the basic element 702 and the spacer 703 is eliminated, and the basic element 702 is removed from the pharynx 40 through the spacer 703 in the direction indicated by arrow 63. Then, the spacer 703 is removed from the pharynx 40 and the artificial respiration tube 80 hangs loosely in the trachea 46.

On account of the absence of the support member 520, with the variant of the instrument according to the invention shown in Figures 22-24E it is possible to move the limbs 715 and 716 with respect to one another in such a manner that the space T between the limbs 715, 716 is made large enough to allow the basic element 702 to pass between the limbs 715, 716.

For this purpose, the instrument 700 is provided with the spreading and locking mechanism 720, by means of which it is possible to move apart or spread the limbs 715, 716 of the spacer 703.

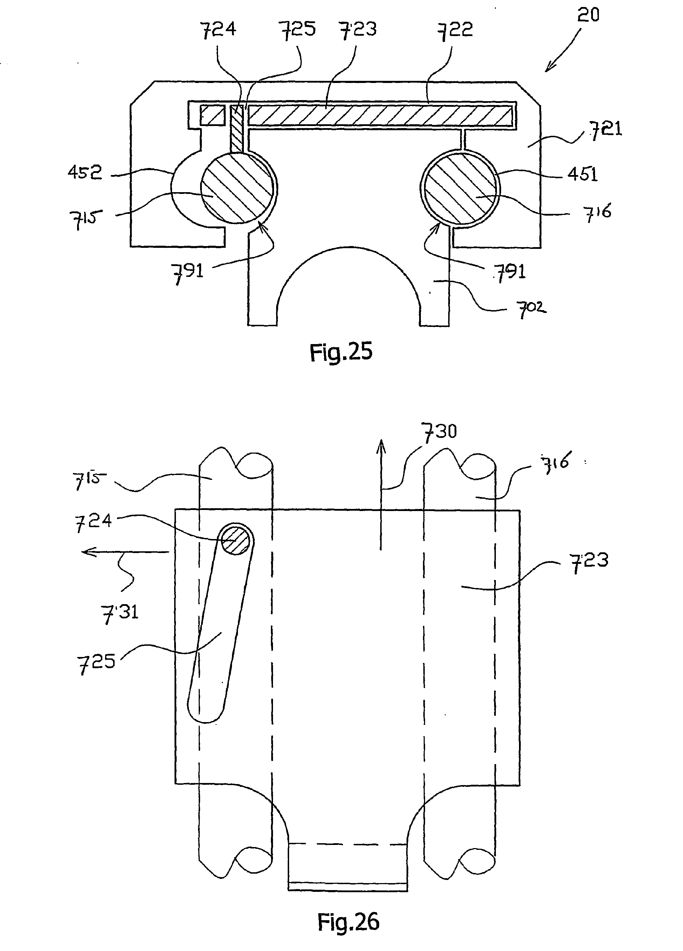

Figure 25 shows a cross section on line IV-IV in Figure 22 through the instrument 700. It can once again be seen from this figure that the basic element 702 is provided at its longitudinal edges with two guide channels 791 which lie opposite one another and in which the limb 715 and the limb 716, respectively, are partially accommodated. The guide channels 791 form a recess in the longitudinal edges of the basic element 702.

The spreading and locking mechanism 720 comprises a housing 721 which, on its sides which face towards the limbs 715 and 716, is provided with first and second guide channels 451 and 452, respectively. At the location of the pin 724, the second guide channel 452 is locally deeper than the first guide channel 451. Furthermore, in the housing 721 there is a receiving space 722 which houses a displaceable actuating element 723. The actuating element 723 is formed as a substantially flat plate. A pin 724 which is directed towards the actuating element 723 and projects partially through a receiving slot 725 provided in the actuating element 723 is also arranged in the limb 715. The limb 716 is preferably fixedly connected to the housing 721 with the aid of connecting means (not shown in more detail).

Figure 26 shows a plan view of a section of the instrument 700, with the housing 721 and the basic element 702 not illustrated. It can clearly be seen that the actuating element 723 with the receiving groove 722 is located above the limbs 715 and 716, and that the pin 724 is located in the receiving slot 725.

If, from the position shown in the figure, the actuating element 723 is moved in a direction indicated by arrow 730, the inclined position of the receiving slot 725 will force the pin 724 in a direction which is indicated by arrow 731, so that in this case the limb 715 on which the pin 724 is arranged will likewise move in the direction of the arrow 731, and the limbs 715 and 716 are spread. In this case, the limb 715 will be pressed into the local deepening of the guide channel 452. Then, the basic element 702 (not shown) can be removed from the spacer 703 without being impeded by the spacer 703.

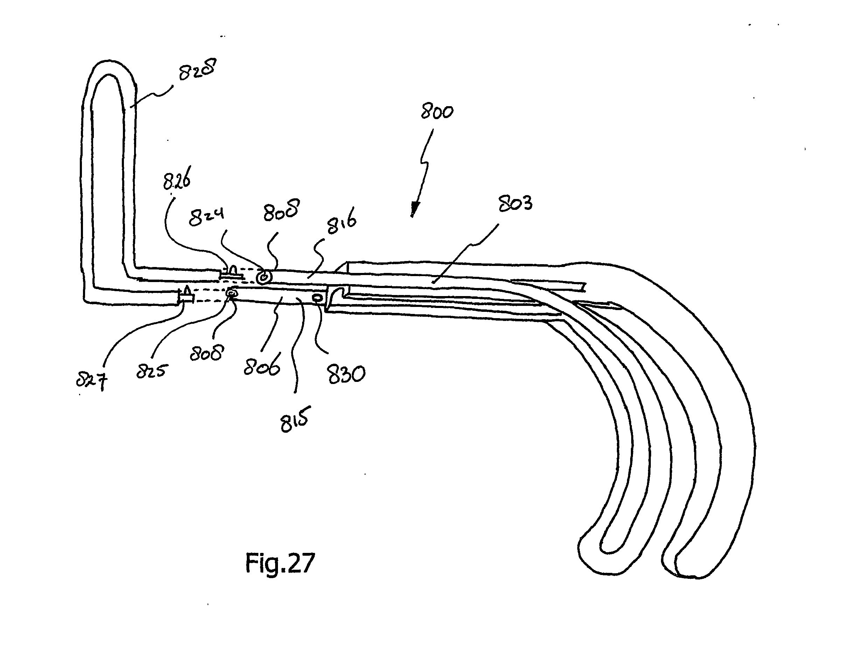

An alternative embodiment of an instrument 800 is shown in Figure 27.

In the highly favourable variant of the instrument 800 shown in Figure 27, the spacer 803 is designed so that it can be split. The spacer 803 is formed from a tubular material, which makes it possible to create two receiving openings 824 and 825 at the two free ends 808 of the straight section 806. These receiving openings 824, 825 can receive corresponding insertion projections 826, 827 which are provided on a handle 828, which in the variant shown is made from the same material as the spacer 803. Additional coupling members can be provided on the insertion projections 826, 827 in order to bring about coupling to the spacer 803. These may, for example, be complementary projection-and-hole connection means.

The major advantage that is achieved with the variant shown compared to the variants shown above is that the spacer 803 and the basic element 802 are very short, so that it is even possible to introduce an artificial respiration tube if the person has his/her chin resting on his/her chest. This may be the case, for example, in the event of a neck injury or another situation in which the person has adopted a twisted posture. After the introduction, it is possible to remove the handle 828 and to allow the remainder of the instrument 800 to rest in the mouth of the person.

A second major advantage is that with the instrument 800 it is possible, for example in cases which require very urgent treatment, for the artificial respiration tube which has been introduced to already have been coupled to the artificial respiration apparatus before the instrument 800 has been removed from the pharynx of the person. This is firstly because the instrument 800 is so compact that it could remain in place without problems and without impeding further actions, and on the other hand because after removal of the handle 828 that end of the spacer 803 which is situated outside the mouth of the person is completely open, unlike with the instrument 700 shown in Figure 22, and therefore the artificial respiration tube which has been introduced can be used without problems.

A highly advantageous way of providing the instrument 800 with locking means for locking the basic element 802 and the spacer 803 in the holding-open position is to provide one or more projections 830, only one of which can be seen in the figure, on that side of the limbs 815, 816 which faces the basic element 802. When the basic element 802 and the spacer 803 are displaced with respect to one another, from the introduction position towards the holding-open position, these projections 830, when they move past the end of the basic element 802, will hook behind this end and prevent displacement back in the other direction.

When the instrument 800 is being removed from the pharynx, the basic element 802 can easily be removed by manually pressing the limbs 815, 816 of the spacer 803 apart.

The second variant of the instrument according to the present invention has been described above with a view to the introduction of a tubular object into the pharynx and in particular into the trachea of a person, it advantageously being possible to adjust the direction in which the said object approaches the trachea. In particular, on account of the very small thickness of the instrument, the trachea can be approached with a very high level of accuracy even in the case of a person who can scarcely open his mouth.

In a number of situations, however, it is desirable for a tubular object to be positioned in the oesophagus instead of in the trachea. In this context, consideration may be given, for example, to the introduction of an ultrasound probe into the oesophagus in order to carry out an ultrasound scan of the heart. In this case, too, the instrument according to the invention can be used very successfully, especially if the coupling means are omitted. This is because, with an instrument according to the invention which has been modified in this way, it is still very easy to introduce the instrument into the pharynx without the person using the instrument having to be able to see where it is located. In addition, the advantage of the small thickness of the instrument remains, so that a person does not have to open his mouth completely. If, for example, in this context the insertion part is provided with a shorter length, a tubular object which is introduced into the pharynx with the aid of the instrument according to the invention will move into the oesophagus when it slides further onwards.

It is also possible, in a similar way to that described above, to provide the insertion part with a "pre-curvature", in which case the insertion part, in a stress-free state, has a curvature which is less than the curvature of the follower part. As a result, moving the insertion part and the follower part apart will lead to the insertion part adopting its "natural" position and thus automatically adopting a less curved position than the follower part. In this way, it is easy for a tubular object which is to be arranged in the oesophagus to be introduced via an insertion part which points further downwards. To allow this solution to be used successfully, with the boundary condition that in the closed position of the instrument the insertion part and the follower part adjoin one another, it is possible to provide click-action means on the insertion part and/or the follower part which, in the closed position, lock the insertion part so that it cannot release its stresses, with the result that the insertion part can only release its stresses when it is extended.

By way of example, it is possible to provide the insertion part with a greater curvature than the follower part, so that the artificial respiration air can be supplied to the trachea in even more targeted fashion.

For example, the instrument cannot only be used to introduce an artificial respiration tube. For example, it is eminently possible, for example, to introduce a fiberscope, in which case the adjustment options offered by the instrument according to the invention allow the viewing direction to be changed with ease.

It is also eminently possible for the framework of the basic element to be of hollow design, in order, for example, for a thin optical fibre, such as a fibre-optic cable, to be fitted therein. In this way, the instrument according to the invention can be used to create a means with which it is possible to look at an angle towards, for example, the vocal cords, which was not hitherto possible without using very expensive optical instruments. Therefore, with an instrument of this type, it is possible to provide someone with a very good view of the trachea and/or the oesophagus from the outside without it being necessary for the person undergoing treatment to have his mouth wide open.

It is also possible to simplify the change in the curvature of the insertion part by weakening the longitudinal edge of the insertion part. This can be done, for example, by cutting into the longitudinal edge.

A further possibility is for the coupling means which are arranged in the vicinity of the ends of the follower part and the insertion part to be designed in such a way that they are releasable, so that the basic element and the spacer can be removed from the pharynx separately from one another. One possible way of doing this is to produce the framework of the basic element from a hollow tube, with an opening being created in each end of the two limbs of the basic element, these openings being in communication with a corresponding opening in the end of the follower part, and two fibres being guided through the framework and fixed to the insertion part.

When the instrument is to be removed from the pharynx, the insertion part can be taken out of the pharynx, with the fibres being pulled through the framework, without the basic element having to come with the insertion part. Moreover, the fibres in the vicinity of the basic element can be pulled, so that the insertion part can be curved.

It is also possible for the basic element to be provided with a closed structure rather than an open structure. In this case, the spacer rests, for example, on the basic element. In order nevertheless to be able to introduce a tubular object into the pharynx along the spacer, it is necessary for there to be a through-opening between the spacer and the basic element in order for the tubular object to be guided through it. It is also possible for a part of the spacer which lies on the actuating side to be located on the lower side of the basic element, in which case at least the straight section thereof projects through the through-opening.

It is also possible for the instrument which is provided with the spreading and locking mechanism to be provided, inside the housing of the spreading and locking mechanism, with locking means which allow the instrument to be opened either in discrete steps or continuously. If locking means of this type are designed, for example, as a resilient lip provided in the housing (or as some other member which is under spring load, for example a ball which is under spring load) and interacting with toothing arranged on the top side of the spacer, effective locking is created, preventing the instrument from undesirably moving back from an open position into a closed position. If, moreover, the resilient lip only acts on one side, i.e. only allows a movement of the spacer towards the open position of the instrument, the instrument can be locked so that it cannot move back out of the open position into the closed position.

Therefore, the invention creates, inter alia, an instrument which is very easy to handle and which enables a tubular object to be arranged in the pharynx, trachea or oesophagus with a very high level of reliability and without the person who is handling the instrument having a clear view of the pharynx. Moreover, the instrument is eminently suitable for use for people who, for whatever reason, cannot open their mouth sufficiently wide, so that there is only a small amount of space between the teeth for the instrument to be introduced. Furthermore, the instrument results in space being created in the pharynx as a result of compressive forces being exerted in the pharynx itself instead of lifting and pushing forces being exerted from outside. This virtually eliminates the risk of injury. Furthermore, the invention creates an instrument which can very easily be removed from the pharynx after a tubular object has been placed in the pharynx, trachea or oesophagus.

## Claims

1. Instrument (1) which is to be introduce into a human pharynx (40) for introducing a tubular object into the trachea or in the oesophagus, comprising:
- a basic element (2) having a curved insertion part (5) with an insertion end (12) which can be introduced into an area between the tongue (41) and the rear side (45) of the pharynx (40), wherein the basic element (2) delimits a guide channel (104a) for guiding the tubular object, wherein the instrument also comprises:
- a spacer (3) which is active in the area between the tongue (41) and the rear side (45) of the pharynx (40), the spacer (3) comprising a curved follower part (5) having an end (11), the follower part (5) in the introduction position of the spacer (3) with respect to the basic element (2), extending substantially along the insertion part (4), and the follower part being adapted to match the contour of the tongue (41), the basic element (2) and the spacer (3), being adapted in the introduction position of the spacer (3) with respect to the basic element (2), to be introduced into the area between the tongue (41) and the rear side (45) of the pharynx (40), and the basic element (2) and the spacer (3), being adapted after they have been introduced, to be displaced away from one another in relative terms in the area between the tongue (41) and the rear side (45) of the pharynx (40), into a holding-open position.

2. Instrument (1) according to claim 1, **characterized in that** the follower part (5) extends beyond the insertion end (12) of the insertion part (4).

3. Instrument (1) according to claim 1 or 2, **characterized in that** the follower part (5) and/or the insertion part (4) extends substantially through an angle of 90°.

4. Instrument (1) according to claim 1, **characterized in that** the follower part (5) and/or insertion part (4) extends through an angle of at least 90°, in particular through an angle of greater than 90°.

5. Instrument (1) according to one of the preceding claims, **characterized in that** the insertion part (4) is at least partially made from a deformable material, in particular an elastically deformable material.

6. Instrument (1) according to one of the preceding claims, **characterized in that** the instrument (1) is provided with locking means for locking the basic element and the spacer at least in the holding-open position.

7. Instrument (1) according to claim 6, **characterized in that** the spacer (3) and the basic element (2) comprise cooperating guide means.

8. Instrument (1) according to one of the preceding claims, **characterized in that** the spacer (3) and the basic element (2) are provided with guide means which engage with one another in complementary fashion.

9. Instrument according to one of the preceding claims, **characterized in that** the basic element (2) and the spacer (3), in the introduction position, substantially adjoin one another and, in the introduction position, have a combined thickness which is substantially equal to the maximum thickness of the basic element (2) and/or the spacer (3).

10. Instrument (1) according to one of the preceding claims, **characterized in that** the spacer (3) comprises at least two limbs (15, 16) which are positioned at a distance (T) from one another.

11. Instrument (1) according to claim 10, **characterized in that** the limbs (15, 16) form an open structure for receiving the basic element (2) in the spacer (3) in order to allow displacement of the basic element (2) through the spacer (3).

12. Instrument (1) according to claim 11, **characterized in that** the basic element (2) comprises guide channels (18) for receiving the limbs (15, 16) in such a manner that they are guided therein.

13. Instrument (1) according to one of the preceding claims, **characterized in that** the basic element (2) delimits a form-fitting through-flow passage (S).

14. Instrument (1) according to one of the preceding claims, **characterized in that** the spacer (3) is provided, at a free end, with a first delimiting body (31) for limiting the insertion depth of the spacer (3).

15. Instrument (1) according to one of the preceding claims, **characterized in that** the basic element (2) is provided, at a free end (13), with a second delimiting body (14) for limiting the insertion depth of the basic element (2).

16. Instrument (1) according to claims 14 and 15, **characterized in that** the locking means are arranged in the vicinity of the delimiting bodies (14, 31).

17. Instrument (1) according to claim 14, **characterized in that** the first delimiting body (31) comprises at least one through-opening for supporting at least a section (7) of the basic element (2) in such a manner that it is guided therein.

18. Instrument (1) according to claim 15, **characterized in that** the second delimiting body (14) comprises a closure plate.

19. Instrument according to claim 1, **characterized in that** the basic element (2) and the insertion part (4) are designed to guide a tubular object (80) over at least a section of an inner surface (104a) of the basic element (2) and the insertion part (4).

20. Instrument according to claim 19, **characterized in that** at least a part of the inner surface (104a) of the basic element (2) is provided with delimiting members (104b), which extend in the longitudinal direction, for forming a guide channel.

21. Instrument according to claim 19 or 20, **characterized in that** in the introduction position of the instrument (1) the insertion part (4) has its inner surface (104a) resting on the follower part (5), and wherein the follower part (5) and the insertion part (4) have substantially the same radius, and the insertion part (4), in the holding-open position of the instrument (1), being located at a distance from the follower part (5), and means being provided for providing the insertion part (4) with a smaller radius than the follower part (5) in the holding-open position.

22. Instrument according to claim 21, **characterized in that** in the closed position the insertion part (104) rests with prestress on the follower part (105).

23. Instrument according to one of claims 19-22, **characterized in that** there are coupling means (571, 572) for limited and displaceable coupling of the basic element (502) and the spacer (503), the coupling means (571, 572) being arranged in the vicinity of a free end (511) of the follower part (505) and in the vicinity of a free end (512) of the insertion part (504).

24. Instrument according to claim 23, **characterized in that** the coupling means (571, 572) are made at least in part from a flexible material.

25. Instrument according to one of claims 19-24, **characterized in that** the spacer (503) comprises a support member (520) which functions as a tilting point for the basic element (502).

26. Instrument according to claim 25, **characterized in that** the basic element (502) bears freely against the support member (520).

27. Instrument according to one of claims 19-26, **characterized in that** the spacer (503) and/or the basic element (502) comprise a substantially straight section (506, 507) which adjoins the follower part (505) and the insertion part (504), respectively.

28. Instrument according to one of claims 19-27, **characterized in that** the spacer (503) and the basic element (502) are provided with gripper means.

29. Instrument according to one of claims 19-28, **characterized in that** the spacer (503) comprises two limbs (515, 516) which are positioned at a distance from one another, for at least partially receiving the basic element (502) between them in the spacer (503).

30. Instrument according to one of claims 19-29, **characterized in that** the spacer (703) and the basic element (702) comprise a substantially straight section (706, 707) which adjoins the follower part (705) and the insertion part (704), respectively, the basic element (702) being provided, over at least a section of the straight section (707), with two guide channels (791) for supporting the spacer (703) in such a manner that it is guided.

31. Instrument according to claim 30, **characterized in that** there is a spreading and locking mechanism (720) for increasing the distance between the limbs (715, 716).

32. Instrument according to claim 31, **characterized in that** the spreading mechanism (720) comprises a housing (721) in which there is a displaceable actuating element (723), which actuating element (723) is formed as a substantially flat plate with a substantially diagonal receiving slot (725) arranged therein, which receiving slot (725) interacts with a pin (724) provided on a limb (715).

## Patentansprüche

1. Instrument (1), das in den Rachen (40) eines Menschen eingeführt werden soll, um ein röhrenförmiges Objekt in die Luftröhre oder in die Speiseröhre einzuführen, das umfasst:
- ein Basiselement (2), das ein gekrümmtes Einsetzteil (5) mit einem Einsetzende (12) besitzt, das in einen Bereich zwischen der Zunge (41) und der Rückseite (45) des Rachens (40) eingeführt werden kann, wobei das Basiselement (2) einen Führungskanal (104a) zum Führen des röhrenförmigen Objekts begrenzt, wobei das Instrument außerdem umfasst:
- einen Abstandshalter (3), der in dem Bereich zwischen der Zunge (41) und der Rückseite (45) des Rachens (40) aktiv ist, wobei der Abstandshalter (3) ein gekrümmtes Folgerteil (5) mit einem Ende (11) aufweist, wobei sich das Folgerteil (5) in der Einführungsposition des Abstandshalters (3) in Bezug auf das Basiselement (2) im Wesentlichen längs des Einsetzteils (4) erstreckt und wobei das Folgerteil dazu ausgelegt ist, sich an den Umriss der Zunge (41) anzupassen, wobei das Basiselement (2) und der Abstandshalter (3) dazu ausgelegt sind, in der Einführungsposition des Abstandshalters (3) in Bezug auf das Basiselement (2) in den Bereich zwischen der Zunge (41) und der Rückseite (45) des Rachens (40) eingeführt zu werden, und wobei das Basiselement (2) und der Abstandshalter (3) dazu ausgelegt sind, nachdem sie eingeführt worden sind, in dem Bereich zwischen der Zunge (41) und der Rückseite (45) des Rachens (40) relativ voneinander weg in eine Aufhalteposition verlagert zu werden.

2. Instrument (1) nach Anspruch 1, **dadurch gekennzeichnet, dass** sich das Folgerteil (5) über das Einsetzende (12) des Einsetzteils (4) hinaus erstreckt.

3. Instrument (1) nach Anspruch 1 oder 2, **dadurch gekennzeichnet, dass** sich das Folgerteil (5) und/oder das Einsetzteil (4) im Wesentlichen über einen Winkel von 90° erstrecken.

4. Instrument (1) nach Anspruch 1, **dadurch gekennzeichnet, dass** sich das Folgerteil (5) und/oder das Einsetzteil (4) über einen Winkel von wenigstens 90°, insbesondere über einen Winkel von mehr als 90° erstrecken.

5. Instrument (1) nach einem der vorhergehenden Ansprüche, **dadurch gekennzeichnet, dass** das Einsetzteil (4) wenigstens teilweise aus einem verformbaren Material, insbesondere aus einem elastisch verformbaren Material hergestellt ist.

6. Instrument (1) nach einem der vorhergehenden Ansprüche, **dadurch gekennzeichnet, dass** das Instrument (1) mit Verriegelungsmitteln versehen ist, um das Basiselement und den Abstandshalter wenigstens in der Aufhalteposition zu verriegeln.

7. Instrument (1) nach Anspruch 6, **dadurch gekennzeichnet, dass** der Abstandshalter (3) und das Basiselement (2) zusammenwirkende Führungsmittel umfassen.

8. Instrument (1) nach einem der vorhergehenden Ansprüche, **dadurch gekennzeichnet, dass** der Abstandshalter (3) und das Basiselement (2) mit Führungsmitteln versehen sind, die auf komplementäre Weise miteinander in Eingriff stehen.

9. Instrument nach einem der vorhergehenden Ansprüche, **dadurch gekennzeichnet, dass** das Basiselement (2) und der Abstandshalter (3) in der Einführungsposition im Wesentlichen aneinander grenzen und in der Einführungsposition eine kombinierte Dicke haben, die im Wesentlichen gleich der maximalen Dicke des Basiselements (2) und/oder des Abstandshalters (3) ist.

10. Instrument (1) nach einem der vorhergehenden Ansprüche, **dadurch gekennzeichnet, dass** der Abstandshalter (3) wenigstens zwei Schenkel (15, 16) umfasst, die in einem gegenseitigen Abstand (T) positioniert sind.

11. Instrument (1) nach Anspruch 10, **dadurch gekennzeichnet, dass** die Schenkel (15, 16) eine offene Struktur bilden, um das Basiselement (2) im Abstandshalter (3) aufzunehmen, um eine Verlagerung des Basiselements (2) durch den Abstandshalter (3) zuzulassen.

12. Instrument (1) nach Anspruch 11, **dadurch gekennzeichnet, dass** das Basiselement (2) Führungskanäle (18) umfasst, um die Schenkel (15, 16) in der Weise aufzunehmen, dass sie darin geführt werden.

13. Instrument (1) nach einem der vorhergehenden Ansprüche, **dadurch gekennzeichnet, dass** das Basiselement (2) einen formangepassten Durchflusskanal (S) begrenzt.

14. Instrument (1) nach einem der vorhergehenden Ansprüche, **dadurch gekennzeichnet, dass** der Abstandshalter (3) an einem freien Ende mit einem ersten Begrenzungskörper (31) versehen ist, um die Einsetztiefe des Abstandshalters (3) zu begrenzen.

15. Instrument (1) nach einem der vorhergehenden Ansprüche, **dadurch gekennzeichnet, dass** das Basiselement (2) an einem freien Ende (13) mit einem zweiten Begrenzungskörper (14) versehen ist, um die Einsetztiefe des Basiselements (2) zu begrenzen.

16. Instrument (1) nach den Ansprüchen 14 und 15, **dadurch gekennzeichnet, dass** die Verriegelungsmittel in der Umgebung der Begrenzungskörper (14, 3 1) angeordnet sind.

17. Instrument (1) nach Anspruch 14, **dadurch gekennzeichnet, dass** der erste Begrenzungskörper (31) wenigstens eine Durchgangsöffnung umfasst, um wenigstens einen Abschnitt (7) des Basiselements (2) in der Weise zu unterstützen, dass er darin geführt wird.

18. Instrument (1) nach Anspruch 15, **dadurch gekennzeichnet, dass** der zweite Begrenzungskörper (14) eine Verschlussplatte umfasst.

19. Instrument nach Anspruch 1, **dadurch gekennzeichnet, dass** das Basiselement (2) und das Einsetzteil (4) so entworfen sind, dass sie ein röhrenförmiges Objekt (80) wenigstens über einen Abschnitt einer inneren Oberfläche (104a) des Basiselements (2) und des Einsetzteils (4) führen.

20. Instrument nach Anspruch 19, **dadurch gekennzeichnet, dass** wenigstens ein Teil der inneren Oberfläche (104a) des Basiselements (2) mit Begrenzungselementen (104b) versehen ist, die sich in der Längsrichtung erstrecken, um einen Führungskanal zu bilden.

21. Instrument nach Anspruch 19 oder 20, **dadurch gekennzeichnet, dass** in der Einführungsposition des Instruments (1) die innere Oberfläche (104a) des Einsetzteils (4) auf dem Folgerteil (5) ruht, wobei das Folgerteil (5) und das Einsetzteil (4) im Wesentlichen den gleichen Radius haben und das Einsetzteil (4) in der Aufhalteposition des Instruments (1) sich in einem Abstand von dem Folgerteil (5) befindet, und Mittel vorgesehen sind, um in der Aufhalteposition für das Einsetzteil (4) einen kleineren Radius als jenen des Folgerteils (5) zu schaffen.

22. Instrument nach Anspruch 21, **dadurch gekennzeichnet, dass** das Einsetzteil (104) in der geschlossenen Position unter Vorbelastung auf dem Folgerteil (105) ruht.

23. Instrument nach einem der Ansprüche 19 bis 22, **dadurch gekennzeichnet, dass** Kopplungsmittel (571, 572) vorgesehen sind, um das Basiselement (502) und den Abstandshalter (503) begrenzt und verlagerbar zu koppeln, wobei die Kopplungsmittel (571, 572) in der Umgebung eines freien Endes (511) des Folgerteils (505) und in der Umgebung eines freien Endes (512) des Einsetzteils (504) angeordnet sind.

24. Instrument nach Anspruch 23, **dadurch gekennzeichnet, dass** die Kopplungsmittel (571, 572) wenigstens teilweise aus einem flexiblen Material hergestellt sind.

25. Instrument nach einem der Ansprüche 19-24, **dadurch gekennzeichnet, dass** der Abstandshalter (503) ein Unterstützungselement (520) umfasst, das als ein Schwenkpunkt für das Basiselement (502) dient.

26. Instrument nach Anspruch 25, **dadurch gekennzeichnet, dass** das Basiselement (502) an dem Unterstützungselement (520) frei anliegt.

27. Instrument nach einem der Ansprüche 19-26, **dadurch gekennzeichnet, dass** der Abstandshalter (503) und/oder das Basiselement (502) einen im Wesentlichen geradlinigen Abschnitt (506, 507) aufweisen, der an das Folgerteil (505) bzw. an das Einsetzteil (504) anstößt.

28. Instrument nach einem der Ansprüche 19-27, **dadurch gekennzeichnet, dass** der Abstandshalter (503) und das Basiselement (502) mit Greifmitteln versehen sind.

29. Instrument nach einem der Ansprüche 19-28, **dadurch gekennzeichnet, dass** der Abstandshalter (503) zwei Schenkel (515, 516) umfasst, die in einem Abstand voneinander positioniert sind, um das Basiselement (502) zwischen ihnen in dem Abstandshalter (503) wenigstens teilweise aufzunehmen.

30. Instrument nach einem der Ansprüche 19-29, **dadurch gekennzeichnet, dass** der Abstandshalter (703) und das Basiselement (702) einen im Wesentlichen geradlinigen Abschnitt (706, 707) aufweisen, der an das Folgerteil (705) bzw. an das Einsetzteil (704) anstößt, wobei das Basiselement (702) wenigstens auf einem Teil des geradlinigen Abschnitts (707) mit zwei Führungskanälen (791) versehen ist, um den Abstandshalter (703) in der Weise zu unterstützen, dass er geführt wird.

31. Instrument nach Anspruch 30, **dadurch gekennzeichnet, dass** ein Spreiz- und Verriegelungsmechanismus (720) vorgesehen ist, um den Abstand zwischen den Schenkeln (715, 716) zu erhöhen.

32. Instrument nach Anspruch 31, **dadurch gekennzeichnet, dass** der Spreizmechanismus (720) ein Gehäuse (721) umfasst, in dem ein verlagerbares Betätigungselement (723) vorhanden ist, wobei das Betätigungselement (723) im Wesentlichen als flache Platte mit einem darin angeordneten im Wesentlichen diagonalen Aufnahmeschlitz (725) ausgebildet ist, wobei der Aufnahmeschlitz (725) mit einem an einem Schenkel (715) vorgesehenen Stift (724) zusammenwirkt.

## Revendications

1. Instrument (1) destiné à être introduit dans un pharynx humain (40) pour l'introduction d'un objet tubulaire dans la trachée ou dans l'oesophage, comprenant :
- un élément de base (2) présentant une partie courbe d'insertion (5) avec une extrémité d'insertion (12) pouvant être introduite dans une zone entre la langue (41) et l'extrémité arrière (45) du pharynx (40), l'élément de base (2) délimitant un canal de guidage (104a) pour guider l'objet tubulaire, l'instrument comprenant également :
- un élément d'écartement (3) qui est actif dans la zone située entre la langue (41) et l'extrémité arrière (45) du pharynx (40), l'élément d'écartement (3) comprenant une partie suiveuse incurvée (5) présentant une extrémité (11), la partie suiveuse (5) dans la position d'introduction de l'élément d'écartement (3) par rapport à l'élément de base (2) s'étendant sensiblement le long de la partie d'insertion (4), et la partie suiveuse étant adaptée à correspondre aux contours de la langue (41), l'élément de base (2) et l'élément d'écartement (3) étant adaptés, dans la position d'introduction de l'élément d'écartement (3) par rapport à l'élément de base (2), à être introduits dans la zone située entre la langue (41) et l'extrémité arrière (45) du pharynx (40), et l'élément de base (2) et l'élément d'écartement (3) étant adaptés, après leur introduction, pour être écartés relativement l'un par rapport à l'autre dans la zone située entre la langue (41) et l'extrémité arrière (45) du pharynx (40), dans une position de maintien d'ouverture.

2. Instrument (1) selon la revendication 1, **caractérisé en ce que** la partie suiveuse (5) s'étend au-delà de l'extrémité d'insertion (12) de la partie d'insertion (4).

3. Instrument (1) selon la revendication 1 ou 2, **caractérisé en ce que** la partie suiveuse (5) et/ou la partie d'insertion (4) s'étend / s'étendent sensiblement à travers un angle de 90°.

4. Instrument (1) selon la revendication 1, **caractérisé en ce que** la partie suiveuse (5) et/ou la partie d'insertion (4) s'étende / s'étendent à travers un angle d'au moins 90°, en particulier à travers un angle supérieur à 90°.

5. Instrument (1) selon l'une quelconque des revendications précédentes, **caractérisé en ce que** la partie d'insertion (4) est au moins partiellement réalisée en un matériau déformable, en particulier un matériau élastiquement déformable.

6. Instrument (1) selon l'une quelconque des revendications précédentes, **caractérisé en ce que** l'instrument (1) est muni de moyen de verrouillage pour verrouiller l'élément de base ainsi que l'élément d'écartement au moins dans la position de maintien d'ouverture.

7. Instrument (1) selon la revendication 6, **caractérisé en ce que** l'élément d'écartement (3) et l'élément de base (2) comprennent des moyens de guidage en coopération.

8. Instrument (1) selon l'une quelconque des revendications précédentes, **caractérisé en ce que** l'élément d'écartement (3) et l'élément de base (2) sont munis de moyens de guidage qui viennent en prise l'un avec l'autre de façon complémentaire.

9. Instrument selon l'une quelconque des revendications précédentes, **caractérisé en ce que** l'élément de base (2) et l'élément d'écartement (3), dans la position d'introduction, se trouvent sensiblement l'un adjacent à l'autre et, dans la position d'introduction, présentent une épaisseur combinée qui est sensiblement égale à l'épaisseur maximale de l'élément de base (2) et/ou de l'élément d'écartement (3).

10. Instrument (1) selon l'une quelconque des revendications précédentes, **caractérisé en ce que** l'élément d'écartement (3) comprend au moins deux branches (15, 16) qui sont positionnées à une distance (T) l'une par rapport à l'autre.

11. Instrument (1) selon la revendication 10, **caractérisé en ce que** les branches (15, 16) forment une structure ouverte pour accueillir l'élément de base (2) dans l'élément d'écartement (3) pour permettre un déplacement de l'élément de base (2) à travers l'élément d'écartement (3).

12. Instrument (1) selon la revendication 11, **caractérisé en ce que** l'élément de base (2) comprend des canaux de guidage (18) pour accueillir les branches (15, 16) d'une manière telle qu'elles soient guidées dans les canaux.

13. Instrument (1) selon l'une quelconque des revendications précédentes, **caractérisé en ce que** l'élément de base (2) délimite un passage d'écoulement (S) à correspondance de forme.

14. Instrument (1) selon l'une quelconque des revendications précédentes, **caractérisé en ce que** l'élément d'écartement (3) est muni, à une extrémité libre, d'un premier corps de délimitation (31) pour limiter la profondeur d'insertion de l'élément d'écartement (3).

15. Instrument (1) selon l'une quelconque des revendications précédentes, **caractérisé en ce que** l'élément de base (2) est muni, à une extrémité libre (13), d'un deuxième corps de délimitation (14) pour limiter la profondeur d'insertion de l'élément de base (2).

16. Instrument (1) selon les revendications 14 et 15, **caractérisé en ce que** les moyens de verrouillage sont agencés à proximité des corps de délimitation (14, 31).

17. Instrument (1) selon la revendication 14, **caractérisé en ce que** le premier corps de délimitation (31) comprend au moins une ouverture de passage pour supporter au moins une section (7) de l'élément de base (2) d'une manière telle qu'il est guidé dans l'ouverture.

18. Instrument (1) selon la revendication 15, **caractérisé en ce que** le deuxième corps de délimitation (14) comprend une plaque de fermeture.

19. Instrument selon la revendication 1, **caractérisé en ce que** l'élément de base (2) et la partie d'insertion (4) sont conçus pour guider un objet tubulaire (80) sur au moins une zone d'une surface intérieure (104a) de l'élément de base (2) et la partie d'insertion (4).

20. Instrument selon la revendication 19, **caractérisé en ce que** au moins une partie de la surface intérieure (104a) de l'élément de base (2) est munie d'organes de délimitation (104b) qui s'étendent selon la direction longitudinale, pour former un canal de guidage.

21. Instrument selon la revendication 19 ou 20, **caractérisé en ce que**, dans la position d'introduction de l'instrument (1), la partie d'insertion (4) présente sa surface intérieure (104a) en appui sur la partie suiveuse (5), et la partie suiveuse (5) et la partie d'insertion (4) ont sensiblement le même rayon, et la partie d'insertion (4), dans la position de maintien d'ouverture de l'instrument (1) se trouvant située à une distance de la partie suiveuse (5), et des moyens étant prévus pour munir la partie d'insertion (4) d'un rayon plus faible que la partie suiveuse (5) dans la position de maintien d'ouverture.

22. Instrument selon la revendication 21, **caractérisé en ce que**, dans la position de fermeture, la partie d'insertion (104) s'appuie avec précontrainte sur la partie suiveuse (105).

23. Instrument selon l'une des revendications 19 à 22, **caractérisé en ce que** sont présents des moyens de couplage (571, 572) pour le couplage limité et déplaçable de l'élément de base (502) et de l'élément d'écartement (503), les moyens de couplage (571, 572) étant agencés à proximité d'une extrémité libre (511) de la partie suiveuse (505) et à proximité d'une extrémité libre (512) de la partie d'insertion (504).

24. Instrument selon la revendication 23, **caractérisé en ce que** les moyens de couplage (571,572) sont réalisées au moins en partie en un matériau flexible.

25. Instrument selon l'une des revendications 19 à 24, **caractérisé en ce que** l'élément d'écartement (503) comprend un organe de support (520) qui fonctionne comme un point de basculement pour l'élément de base (502).

26. Instrument selon la revendication 25, **caractérisé en ce que** l'élément de base (502) s'appuie librement contre l'organe de support (520).

27. Instrument selon l'une des revendications 19 à 26, **caractérisé en ce que** l'élément d'écartement (503) et/ou l'élément de base (502) comprend / comprennent une zone sensiblement rectiligne (506, 507) qui est adjacente respectivement à la partie suiveuse (505) et la partie d'insertion (504).

28. Instrument selon l'une des revendications 19 à 27, **caractérisé en ce que** l'élément d'écartement (503) et l'élément de base (502) sont munis de moyens de préhension.

29. Instrument selon l'une des revendications 19 à 28, **caractérisé en ce que** l'élément d'écartement (503) comprend deux branches (515, 516) qui sont positionnées à une distance l'une de l'autre, pour recevoir au moins en partie l'élément de base (502) entre elles dans l'élément d'écartement (503).

30. Instrument selon l'une des revendications 19 à 29, **caractérisé en ce que** l'élément d'écartement (703) et l'élément de base (702) comprennent une zone sensiblement rectiligne (706, 707) qui est adjacente à la partie suiveuse (705) et respectivement, la partie d'insertion (704), l'élément de base (702) étant muni, sur au moins une zone de la zone rectiligne (707), de deux canaux de guidage (791) pour supporter l'élément d'écartement (703) d'une manière telle qu'il soit guidé.

31. Instrument selon la revendication 30, **caractérisé par** la présence d'un mécanisme d'élargissement et de blocage (720) pour augmenter la distance entre les branches (715, 716).

32. Instrument selon la revendication 31, **caractérisé en ce que** le mécanisme d'élargissement (720) comprend un boîtier (721) dans lequel est situé un organe d'actionnement déplaçable (723), cet organe d'actionnement (723) étant réalisé sous forme d'une plaque sensiblement plane avec une fente d'accueil sensiblement diagonale (725) agencée en son sein, cette fente d'accueil (725) interagissant avec une broche (724) prévue sur une branche (715).
